(19) Europäisches Patentamt
European Patent Office
Office européen des brevets

(11) EP 4 188 910 B1

(12) # EUROPEAN PATENT SPECIFICATION

(45) Date of publication and mention of the grant of the patent:
**23.07.2025 Bulletin 2025/30**

(21) Application number: **21762358.6**

(22) Date of filing: **30.07.2021**

(51) International Patent Classification (IPC):
***C07D 221/02*** $^{(2006.01)}$ ***C07D 311/02*** $^{(2006.01)}$
***C07D 335/04*** $^{(2006.01)}$ ***C07C 35/32*** $^{(2006.01)}$
***C07C 49/633*** $^{(2006.01)}$ ***C07C 325/02*** $^{(2006.01)}$
***A01N 31/06*** $^{(2006.01)}$ ***A01N 35/06*** $^{(2006.01)}$
***A01N 43/16*** $^{(2006.01)}$ ***A01N 43/18*** $^{(2006.01)}$
***A01N 43/42*** $^{(2006.01)}$ ***G01N 33/00*** $^{(2006.01)}$

(52) Cooperative Patent Classification (CPC):
**C07D 221/02; A01N 31/06; A01N 33/04; A01N 35/06; A01N 43/16; A01N 43/18; A01N 43/42; A01P 7/00; C07C 325/02; C07D 311/02; C07D 335/04**

(86) International application number:
**PCT/EP2021/071500**

(87) International publication number:
**WO 2022/023572 (03.02.2022 Gazette 2022/05)**

(54) **COMPOUNDS WITH SEMIOCHEMICAL PROPERTIES AND BIOSENSORS**

VERBINDUNGEN MIT SEMIOCHEMISCHEN EIGENSCHAFTEN UND BIOSENSOREN

COMPOSÉS AYANT DES PROPRIÉTÉS SÉMIOCHIMIQUES ET BIOCAPTEURS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.07.2020 GB 202011989**

(43) Date of publication of application:
**07.06.2023 Bulletin 2023/23**

(73) Proprietor: **Rothamsted Research Limited**
**Hertfordshire AL5 2JQ (GB)**

(72) Inventors:
- **SIMS, Cassie Marie**
  **Harpenden, Hertfordshire AL5 2JQ (GB)**
- **BIRKETT, Mike**
  **Harpenden, Hertfordshire AL5 2JQ (GB)**
- **WITHALL, David**
  **Harpenden, Hertfordshire AL5 2JQ (GB)**
- **POXON, Alistair**
  **Harpenden, Hertfordshire AL5 2JQ (GB)**

(74) Representative: **Marks & Clerk LLP**
**15 Fetter Lane**
**London EC4A 1BW (GB)**

(56) References cited:
**WO-A1-2006/072037 WO-A1-2006/072039**
**WO-A2-2006/096876 WO-A2-2007/133683**

- **—- —- –-: "Annex of SA1866438", –-, 1 January 1996 (1996-01-01), XP055864245, Retrieved from the Internet <URL:—> [retrieved on 20211122]**
- **YUFENG SUN ET AL: "New Analogues of ( E )-&bgr;-Farnesene with Insecticidal Activity and Binding Affinity to Aphid Odorant-Binding Proteins", JOURNAL OF AGRICULTURAL AND FOOD CHEMISTRY, vol. 59, no. 6, 23 March 2011 (2011-03-23), pages 2456 - 2461, XP055029923, ISSN: 0021-8561, DOI: 10.1021/jf104712c**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **DE BIASIO FILOMENA ET AL: "Expression pattern analysis of odorant-binding proteins in the pea aphid Acyrthosiphon pisum : OBP expression patterns in the pea aphid", INSECT SCIENCE, vol. 22, no. 2, 1 April 2015 (2015-04-01), United Kingdom, Taiwan, Republic of China, pages 220 - 234, XP055918835, ISSN: 1672-9609, DOI: 10.1111/1744-7917.12118**
- **PAOLO PELOSI ET AL: "Odorant-Binding Proteins as Sensing Elements for Odour Monitoring", SENSORS, vol. 18, no. 10, 27 September 2018 (2018-09-27), pages 1 - 19, XP055758614, DOI: 10.3390/s18103248**
- **PELOSI PAOLO ET AL: "From radioactive ligands to biosensors: binding methods with olfactory proteins", APPLIED MICROBIOLOGY AND BIOTECHNOLOGY, SPRINGER BERLIN HEIDELBERG, BERLIN/HEIDELBERG, vol. 102, no. 19, 27 July 2018 (2018-07-27), pages 8213 - 8227, XP036591459, ISSN: 0175-7598, [retrieved on 20180727], DOI: 10.1007/S00253-018-9253-5**


Remarks:

The complete document including Reference Table(s) and the Sequence Listing(s) can be downloaded from the EPO website

## Description

## BACKGROUND OF THE INVENTION

### 1. Field of the Invention

Provided herein are biosensors for detecting an analyte in a sample, uses of said biosensor, and in vitro methods for detecting an analyte in a sample.

### 2. Related Art

A semiochemical is a compound that is secreted by organisms, which modifies the behaviour and/or development of another organism. Semiochemicals are categorised into intraspecific semiochemicals, *pheromones,* a compound or group of compounds that are released by an organism and induce a response in an individual of the same species, and interspecific semiochemicals, *allelochemicals,* which stimulate organisms of different species. Pheromones are critical for communication between insects of the same species; these may include sex pheromones, aggregation pheromones, and alarm pheromones.

Pheromones can be further categorised into *releasers,* pheromones which induces an immediate behavioural change, and *primers,* pheromones which initiate a complex set of physiological or developmental changes, but may result in no immediate behavioural change.

Semiochemicals are used in host location, mating and enemy warning systems. Although semiochemicals are widely employed by insects for communication, communication chemistry and potential semiochemicals have been identified in many other organisms including mammals, birds and fish.

Aphid species, including the pea aphid, *Acyrthosiphon pisum,* have been shown to employ sex pheromones (e.g. (4a*S*,7*S*,7a*R*)-nepetalactone and (1*R*,4*aS*,7*S*,7*aR*)-nepetalactol) and an alarm pheromone, in addition to a range of allelochemistry generally utilised for host-location.

Semiochemicals have the potential to be used in pest management, by using mating disruption, pheromone traps, push-pull strategies and recruitment of natural enemies. Synthetic sex pheromone components have been used to catch male aphids and recruit foraging parasitic wasps, *Aphidius ervi* Haliday and *Praon barbatum* Mackauer, in the field. The synthetic sex pheromone components have also been found to attract other aphid natural predators, such as lacewings (*Chrysopa cognata).*

The alarm pheromone (*E*)-β-farnesene (EBF) has been shown to be repellent to aphids in behavioural studies, whilst attractive to natural enemy predators and parasitoids. A hexaploid commercial variety of wheat, *Triticum aestivum* cv. Cadenza (Poaceae), has been genetically engineered to biosynthesise and release EBF. Evaluation in field trials showed the transformed wheat variety was not significantly different from non-transformed varieties in managing aphids (assessed by aphid numbers and number of parasitized aphids), although controlled environment studies had demonstrated its effectiveness. This is likely due to the release rate of the alarm pheromone from the plant being consistent and steady, in contrast to a natural quick burst of pheromone produced by aphids.

Plant-derived semiochemicals also have practical applications; (*Z*)-jasmone has been found to be effective in reducing aphid numbers, attracting parasitic wasps and inducing the production of repellent volatiles in crops such as wheat, cotton and sweet peppers. (*S*)-Germacrene D has been identified as a potent repellent for aphids, but has little potential for commercial application in crop protection due to its chemical instability and cost of production. Development of more stable analogues which have comparable behavioural activity could be developed, particularly using modified terpene synthases and unnatural substrates.

Further advancement of the use of semiochemicals in pest management could stem from a deeper understanding of molecular recognition processes in the olfactory systems of pests. This may lead to *'ab initio'* design of ligands, which may have similar behavioural effects as other olfactory ligands, but better prospects for commercial producibility. Currently, understanding of the olfactory system is limited, though two major groups of proteins are involved - olfactory receptors (ORs) and odorant binding proteins (OBPs) - both of which could provide potential pest management targets.

Given the significant value in understanding olfactory systems and semiochemicals in various industries, such as the food, health and pharmaceutical industries, there exists a need to develop new semiochemicals and sensors. The present invention addresses this need.

Sun et al. in "New Analogues of (E)-β-Farnesene with Insecticidal Activity and Binding Affinity to Aphid Odorant-Binding Proteins", Journal of Agricultural and Food Chemistry, 2011, vol. 59, no. 6, pp. 2456-2461, describe analogues of (E)-β-Farnesene that bind to odorant-binding proteins of the pea aphid *Acyrthosiphon pisum.*
de Biasio et al. in "Expression pattern analysis of odorant-binding proteins in the pea aphid Acyrthosiphon pisum", Insect Science, 2015, vol. 22, no. 2, pp. 220-234, describe relations between *Acyrthosiphon pisum* odorant-binding protein expression patterns and their functional implications.

Pelosi et al. in "Odorant-Binding Proteins as Sensing Elements for Odour Monitoring", Sensors, 2018, vol. 18, no. 10, pp. 1-19, review odorant-binding proteins and other soluble proteins as detecting elements in biosensors for odours.
Pelosi et al. in "From radioactive ligands to biosensors: binding methods with olfactory proteins", Applied Microbiology and Biotechnology, 2018, vol. 102, no. 19, pp. 8213-8227, review binding protocols to measure the affinity of odorants and pheromones to soluble olfactory proteins, such as odorant-binding proteins, chemosensory proteins and Niemann-Pick class C2 proteins.

**SUMMARY**

The invention relates generally to biosensors for detecting an analyte in a sample.
In an aspect of the invention, there is provided a biosensor as recited in Claim 1, the biosensor comprising:

a protein having an amino acid sequence as defined in SEQ ID NO: 6, or or variant thereof, wherein the variant has at least 80% overall sequence identity to SEQ ID NO: 6; and
a signal generator, wherein the signal generator is configured to output a signal when the analyte is bound to the protein.

Preferably, the biosensor further comprises:

a flow path for moving the sample;
a substrate; and
a protein-containing layer immobilised to the substrate and in contact with the flow path, wherein the protein-containing layer comprises the protein.

In another aspect of the invention, there is provided use of the biosensor, wherein the biosensor is used to identify olfactory ligands.
In another aspect of the invention, there is provided use of the biosensor, wherein the biosensor is used in high-throughput screening.
In another aspect of the invention, there is provided use of the biosensor, wherein the biosensor is used to detect field populations of aphids.
In another aspect of the invention, there is provided an in vitro method for detecting an analyte in a sample as recited in Claim 4, the method comprising:

a. providing a biosensor as defined above;
b. contacting the biosensor with the sample; and
c. comparing a magnitude of the signal generated by the biosensor when the sample is present with a reference magnitude of the signal generated by the biosensor when the sample is absent.

Preferably, the biosensor is used to identify olfactory ligands.
Preferably, the biosensor is used in high-throughput screening.
Preferably, the biosensor is used to detect field populations of aphids.

**BRIEF DESCRIPTION OF THE DRAWINGS**

The invention is further described by reference to the following non-limiting figures.

FIG. 1 shows homology models of *A. pisum* odorant binding proteins (OBP1-OBP11).
FIG. 2 shows: (i) predicted structure of OBP6 (green) with potential binding pockets shown in purple; (ii) potential salt bridge between Lys144 and Asp174; (iii) predicted structure of OBP6 with the adaptive Poisson-Boltzmann Solver (APBS) electrostatic map displayed.
FIG. 3 shows results from Autodock ligand-screening. The aphid sex pheromone components and respective enantiomers were tested, along with the aphid alarm pheromone.
FIG. 4 shows predicted binding interactions (shown as $1/K_i$) of the aphid sex pheromone components with OBPs 6-9. The natural enantiomers are indicated with an asterisk.
FIG. 5 shows: (i) OBP6 (green) interactions with the enantiomers of the sex pheromone; naturally occurring (1*R*,4a*S*,7*S*,7a*R*)-nepetalactol (pink) interacting with Tyr176 and Phe208 (red, dashed); (ii) non-naturally occurring (1*S*,4a*R*,7*R*,7a*S*)-nepetalactol (blue) with a potential interaction with Lys169 (red, dashed). Distance of interactions are in Å.

FIG. 6 shows: (i) the general transmembrane domains of insect odorant receptors; (ii) homology models of *Acyrthosiphon pisum* odorant-receptor 5 embedded in a lipid bilayer.

FIG. 7 shows tryptophan fluorescence of OBP6 at 2 μM with 2 μM 1-NPN titrated with various ligands to final concentrations of 0-22 μM. The lowest concentration of each ligand (0 μM) can be seen in red and the highest concentration (22 μM) in black. For each ligand, only one data set is presented for clarity.

FIG. 8 shows: (i) binding curves of OBP6 with the aphid sex pheromone components (black) and their respective enantiomers (red); (ii) binding curves of OBP6 with the aphid alarm pheromone (blue) and linalool (purple); (iii) calculated $K_D$ values of OBP6 with various ligands.

FIG. 9 shows the calculated $K_D$ values for OBP6 and various ligands from fluorescent binding studies versus the predicted $K_D$s from *in silico* testing.

FIG. 10 shows results from Autodock ligand-screening. The aphid sex pheromone components and respective enantiomers were tested, along with the aphid alarm pheromone.

FIG. 11 shows the saturation transfer difference (STD) nuclear magnetic resonance (NMR) spectra of bovine serum albumin (BSA) with tryptophan (7.00-8.00 ppm) and sucrose (3.00-4.40 ppm). The initial $^1H$ can be seen (top spectrum), in addition to the STD NMR (bottom spectrum), where only tryptophan peaks remain.

FIG. 12 shows the STD NMR spectra of *A. pisum* OBP6 with (4aS,7S,7aR)-nepetalactone. The initial $^1H$ spectra (top spectrum), in addition to the STD NMR (bottom spectrum).

FIG. 13 shows: (i) assignments and changes in relative intensity of different peaks in the STD NMR spectrum of OBP6 and (4aS,7S,7aR)-nepetalactone; (ii) STD absolute values and changes in relative intensity of different protons in the STD NMR spectrum of OBP6 and (4aS,7S,7aR)-nepetalactone; (iii) the structure of (4aS,7S,7aR)-nepetalactone annotated with the epitope mapping results.

## DETAILED DESCRIPTION

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

The practice of the present invention will employ, unless otherwise indicated, conventional techniques of microbiology, tissue culture, molecular biology, chemistry, biochemistry, recombinant DNA technology and biosensor fabrication, which are generally known in the art. Such techniques are explained fully in the literature.

### 3. Olfactory Proteins

Olfactory proteins are responsible for the detection of odorants and may therefore be suitable for the detection of sex pheromones associated with insects, for example aphids.

Olfactory proteins may include odorant receptors (ORs). Non-limiting examples of odorant receptors include odorant receptor 1/olfactory receptor co-receptor (OR1/ORCO), odorant receptor 2 (OR2), odorant receptor 4 (OR4), odorant receptor 5 (OR5), odorant receptor 10 (OR10), odorant receptor 17 (OR17), odorant receptor 20 (OR20), odorant receptor 22c (OR22c), odorant receptor 23 (OR23), odorant receptor 25 (OR25), odorant receptor 31 (OR31), odorant receptor 37 (OR37), odorant receptor 38 (OR38),odorant receptor 39 (OR39), odorant receptor 42 (OR42) and odorant receptor 43 (OR43).

Olfactory proteins may include olfactory binding proteins (OBPs). Non-limiting examples of olfactory binding proteins include olfactory binding protein 1 (OBP1), olfactory binding protein 2 (OBP2), olfactory binding protein 3 (OBP3), olfactory binding protein 4 (OBP4), olfactory binding protein 5 (OBP5), olfactory binding protein 6 (OBP6), olfactory binding protein 7 (OBP7), olfactory binding protein 8 (OBP8), olfactory binding protein 9 (OBP9), olfactory binding protein 10 (OBP10) and olfactory binding protein 11 (OBP11).

### 4. Biosensors

The biosensor comprises *Acyrthosiphon pisum* OBP6 (having an amino acid sequence as defined in SEQ ID NO: 6), or a variant thereof.

The term "variant" or "functional variant" as used herein with reference to any of the sequences described herein refers to a variant polypeptide sequence or part of the polypeptide sequence which retains the biological function of the full non-variant sequence. A functional variant also comprises a variant of the polypeptide of interest, which has sequence alterations that do not affect function, for example in non-conserved residues. Also encompassed is a variant that is substantially identical, i.e. has only some sequence variations, for example in non-conserved residues, compared to the wild type sequences as shown herein and is biologically active. Alterations in a polypeptide sequence that does not affect

the functional properties of the polypeptide are well known in the art. For example, the amino acid alanine, a hydrophobic amino acid, may be substituted by another less hydrophobic residue, such as glycine, or a more hydrophobic residue, such as valine, leucine, or isoleucine. Similarly, changes which result in substitution of one negatively charged residue for another, such as aspartic acid for glutamic acid, or one positively charged residue for another, such as lysine for arginine, can also be expected to produce a functionally equivalent product. Each of the proposed modifications is well within the routine skill in the art, as is determination of retention of biological activity of the encoded products.

As used in any aspect described herein, a "variant" or a "functional variant" has at least 80% (for example 81%, 82%, 83%, 84%, 85%, 86%, 87%, 88%, 89%, 90%, 91%, 92%, 93%, 94%, 95%, 96%, 97%, 98%, or at least 99%) overall sequence identity to the non-variant amino acid sequence.

Preferably, a "variant" or a "functional variant" as described herein has at least 80% (for example at least 85%, 90%, 95% or 99%) overall sequence identity to the non-variant amino acid sequence.

It is particularly preferred that a "variant" or a "functional variant" as described herein has at least 90%, 95% or 99% overall sequence identity to the non-variant amino acid sequence.

Two polypeptides are said to be "identical" if the sequence of amino acid residues, respectively, in the two sequences is the same when aligned for maximum correspondence as described below. The terms "identical" or percent "identity", in the context of two or more polypeptide sequences, refer to two or more sequences or subsequences that are the same or have a specified percentage of amino acid residues that are the same, when compared and aligned for maximum correspondence over a comparison window, as measured using one of the following sequence comparison algorithms or by manual alignment and visual inspection. When percentage of sequence identity is used in reference to proteins or peptides, it is recognised that residue positions that are not identical often differ by conservative amino acid substitutions, where amino acid residues are substituted for other amino acid residues with similar chemical properties (e.g., charge or hydrophobicity) and therefore do not change the functional properties of the molecule. Where sequences differ in conservative substitutions, the percent sequence identity may be adjusted upwards to correct for the conservative nature of the substitution. Means for making this adjustment are well known to those of skill in the art. For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters. Non-limiting examples of algorithms that are suitable for determining percent sequence identity and sequence similarity are the BLAST and BLAST 2.0 algorithms.

In an embodiment, the biosensor may further comprise a signal generator. For example, the signal generator may be a transducer.

The signal generator may be configured to output a signal when the analyte has bound to the olfactory protein.

The signal generator may be configured to output a different signal when the analyte is not bound to the olfactory protein.

The signal generator may be configured to output no signal when the analyte is not bound to the olfactory protein.

A skilled person would also understand that a situation where the analyte has not bound to the olfactory protein leads to an output of a signal, and where the analyte has bound to the olfactory protein leads to an output of no signal, would be embraced by the present disclosure.

A magnitude of the signal generated may be correlated or proportional to the concentration of analyte in the sample. For example, a higher concentration of analyte may be associated with a more intense signal in terms of magnitude; conversely, a lower concentration of analyte may be associated with a less intense signal in terms of magnitude.

The signal may be based on an electrical signal. For example, the signal may be a potential difference, a current, a resistance, a capacitance or an impedance associated with the electrical signal.

The signal may be based on an optical signal. For example, the signal may be based on various properties of electromagnetic radiation, such as wavelength, intensity, absorption, scattering or fluorescence. For example, the electromagnetic radiation may be ultraviolet light, visible light or infrared light.

The biosensor may operate based on electrochemical means. Binding of an analyte may cause consumption or generation of electrons. Alternatively, binding of an analyte does not cause direct electron flow but causes changes on an electrode surface, for example, changes in charge, hydration or pH.

Non-limiting examples of an electrochemical-based biosensor include impedimetric, amperometric, potentiometric, conductometric and voltametric biosensors. An electrochemical-based biosensor may include biosensor field-effect transistors (BioFETs), such as ion-sensitive field-effect transistors (ISFETs).

The biosensor may operate based on optical means. For example, the biosensor may operate in a label-free mode, where an optical signal is generated directly by the interaction of the analyte with a transduction element. Alternatively, the biosensor may operate with a label and an optical signal is generated by a colorimetric, fluorescent

or luminescent method.

Non-limiting examples of an optical-based biosensor include surface plasmon resonance (SPR), evanescent wave fluorescence, optical waveguide interferometric, ellipsometric, reflectometric interference spectroscopy (RIfS) and surface-enhanced Raman scattering (SERS) biosensors.

The biosensor may operate based on piezoelectric means. For example, the biosensor may include a piezoelectric crystal (e.g. quartz) which vibrates under the influence of an electric field. The resonant frequency may change as an analyte adsorbs or desorbs from the surface of the piezoelectric crystal.

In an embodiment, the biosensor may further comprise a flow path for moving a sample, wherein the sample may contain an analyte.

In an embodiment, the biosensor may further comprise a substrate.

For example, the substrate may be a glass substrate or a polymer substrate. The substrate may exhibit excellent mechanical strength, thermal stability, transparency, surface smoothness, ease of handling, and water resistance.

In an embodiment, the biosensor may further comprise a protein-containing layer immobilised to the substrate, wherein the protein-containing layer contains the olfactory protein. The protein-containing layer may be in contact with the flow path.

Means for immobilising proteins onto a substrate are well known in the art. Non-limiting methods of immobilising proteins include adsorption, cross-linking, covalent bonding, electrochemical polymerisation and photopolymerisation.

In an embodiment, the biosensor may further comprise a working electrode.

In an embodiment, the biosensor may further comprise a counter electrode.

The working electrode and/or the counter electrode may be partially exposed at the flow path and utilised for applying a voltage to a sample.

The working electrode and/or the counter electrode may comprise an electrically conductive material. An electrically conductive material is any material that allows the flow of charge (electrical current) in one or more directions. Non-limiting examples of electrically conductive materials may include metals and metal alloys, such as gold, platinum, copper, silver, aluminium, palladium, steel, brass and bronze; carbon-based materials, such as graphite, single-wall carbon nanotubes (SWCNTs), multi-wall carbon nanotubes (MWCNTs), graphene, graphene oxide; metal oxides, such as indium tin oxide (ITO), indium zinc oxide (IZO), tin oxide, zinc oxide; and organic conducting polymers, such as polythiophene, polyaniline and polypyrrole.

In an embodiment, the biosensor may further comprise a reference electrode.

The reference electrode may provide a reference signal, for example, a stable potential, relative to the sample. For example, a reference electrode may be an Ag/AgCl electrode.

Biosensors as described herein may have high binding affinity to the sex pheromones of insects and therefore may be highly sensitive to the sex pheromones of insects.

For example, biosensors as described herein may be highly sensitive to the sex pheromones of aphids, and in particular, *Acyrthosiphon pisum.*

Biosensors as described herein may be highly sensitive to the presence of nepetalactols or nepetalactones.

For example, biosensors as described herein may be highly sensitive to the presence of (1*R*,4a*S*,7*S*,7a*R*)-nepetalactol ((1*R*,4a*S*,7*S*,7a*R*)-4,7-dimethyl-1,4a,5,6,7,7a-hexahydrocyclopenta[*c*]pyran-1-ol), (4a*S*,7*S*,7a*R*)-nepetalactone ((4a*S*,7*S*,7a*R*)-4,7-dimethyl-5,6,7,7a-tetrahydrocyclopenta[c]pyran-1(4a*H*)-one), (1*S*,4a*R*,7*R*,7a*S*)-nepetalactol ((1*S*,4a*R*,7*R*,7a*S*)-4,7-dimethyl-1,4a,5,6,7,7a-hexahydrocyclopenta[*c*]pyran-1-ol) and/or (4a*R*,7*R*,7a*S*)-nepetalactone ((4a*R*,7*R*,7a*S*)-4,7-dimethyl-5,6,7,7a-tetrahydrocyclopenta[c]pyran-1(4a*H*)-one).

In particular, the biosensors as described herein may be highly sensitive to the presence of natural nepetalactols or nepetalactones, such as (1*R*,4a*S*,7*S*,7a*R*)-nepetalactol and (4a*S*,7*S*,7a*R*)-nepetalactone.

As used herein, the term "highly sensitive" may refer to a biosensor comprising an olfactory protein as described herein, or a variant thereof, having a binding energy of -7.0 kcal $mol^{-1}$, preferably -7.1 kcal $mol^{-1}$, more preferably -7.2 kcal $mol^{-1}$, even more preferably -7.3 kcal $mol^{-1}$, yet even more preferably -7.4 kcal $mol^{-1}$, most preferably -7.5 kcal $mol^{-1}$, to a nepetalactol or nepetalactone as described herein.

## 5. Uses of Biosensors and Methods for Detecting Analytes

Biosensors as described herein may generally be used for the detection of analytes.

In an embodiment, the biosensors described herein may be used to identify new olfactory ligands.

For example, the new olfactory ligands may be associated with aphids, and in particular, *Acyrthosiphon pisum.*

For example, the biosensor may be used as a lab screening tool to identify new olfactory ligands. For example, the biosensor may be used to identify aphid attractants other than sex pheromone components.

In an embodiment, the biosensors described herein may be used in high-throughput screening.

High-throughput screening (HTS) typically uses automated assays to search through large numbers of compounds for a desired activity. High throughput methods enable researchers to assay thousands of different chemicals

against each target molecule very quickly using robotic handling systems and automated analysis of results.

As used herein, "high throughput screening" or "HTS" refers to the rapid in vitro screening of large numbers of compounds (libraries); generally tens to hundreds of thousands of compounds, using robotic screening assays. Ultra high-throughput screening (uHTS) generally refers to the high-throughput screening accelerated to greater than 100,000 tests per day.

To achieve high-throughput screening, it is advantageous to house samples on a multicontainer carrier or platform. A multicontainer carrier facilitates measuring reactions of a plurality of candidate compounds simultaneously. Multi-well microplates may be used as the carrier. Such multi-well microplates, and methods for their use in numerous assays, are both known in the art and commercially available.

In an embodiment, the biosensors described herein may be used to detect field populations of aphids.

For example, the biosensor may be deployed as a field tool.

In an embodiment, an in vitro method of detecting an analyte in a sample is provided, comprising: (a) providing a biosensor as described herein; (b) contacting the biosensor with the sample; and (c) comparing a magnitude of the signal generated by the biosensor when the sample is present with a reference magnitude of the signal generated by the biosensor when the sample is absent.

## EXPERIMENTAL

The following non-limiting examples are provided for further illustration.

### Examples 1 to 22 (reference): Ligand-Protein Modelling and Docking

Sequence Data and Protein Models

All previously published protein structures were accessed from the Protein Data Bank (PDB).

**Table 1:** NCBI accession codes for *Acyrthosiphon pisum* olfactory proteins.

| ***A. Pisum* Protein** | **Residues** | **NCBI Ascension Code** |
|---|---|---|
| OBP1 | 159 aa | NP_001153526.1 |
| OBP2 | 243 aa | NP_001153528.1 |
| OBP3 | 141 aa | NP_001153529.1 |
| OBP4 | 193 aa | NP_001153530.1 |
| OBP5 | 221 aa | NP_001153531.1 |
| OBP6 | 160 aa | NP_001153532.1 |
| OBP7 | 155 aa | NP_001153533.1 |
| OBP8 | 162 aa | NP_001153534.1 |
| OBP9 | 165 aa | NP_001153535.1 |
| OBP10 | 143 aa | NP_001153525.1 |
| OBP11 | 141 aa | XP_008178459.1 |
| OR1 (ORCO) | 463 aa | AQS60741.1 |
| OR2 | 403 aa | AQS60742.1 |
| OR4 | 368 aa | ARJ54248.1 |
| OR5 | 367 aa | KX890157.1 |
| OR10 | 369 aa | AQS60745.1 |
| OR17 | 430 aa | AQS60746.1 |
| OR20 | 420 aa | AQS60747.1 |
| OR22c | 403 aa | XP_003245950.2 |
| OR39 | 426 aa | AQS60753.1 |

Sequence Alignment and Transmembrane Domain Prediction

Sequence alignments were performed using Cluster Omega and the PRALINE server. Conservation mapping was performed using ConSurf. Transmembrane domain predications of the receptor proteins were performed using a consensus approach and four different serves - HMMTOP, TMpred, PHOBIUS and TMHMM. Alignments and conservation maps were analysed in GeneDoc, and phylogenetic trees were generated in FigTree v1.4.3.

For odorant-binding proteins, protein structures were initially predicted using the iTASSER database, which takes a hierarchical approach by identifying structural templates from the Protein Data Bank. All predicted protein structures were minimised using the Yasara minimisation server.

For odorant-receptors, Homology based modelling was performed using the olfactory receptor co-receptor, ORCO, from *Apocrypta bakeri* as a template. The structure of AbakORCO was obtained from the Protein Data Bank (PDB ID 6C70; resolution 3.5 Å). Pairwise sequence alignment was performed using PRALINE and the predicted transmembrane domains were manually aligned and annotated. The available ORCO structure shares a generally low sequence identity with the *A. pisum* odorant receptors, however, the general seven transmembrane structure should be conserved. The pairwise alignment served as a template for homology modelling using MODELLER 9.3 with loop refinement. The secondary structure of long extracellular loops 2 and 3 (EL2 and EL3) were individually predicted using the iTASSER server. Approximately 25 models were generated for each protein, and these were subsequently assessed using discrete optimised protein energy (DOPE) from MODELLER, in addition to Ramachandran and Z-score analysis, performed in PROCHECK and ProSA respectively.

All protein structures were visualised in PyMol 2.3.4.

Ligand Screening

Ligands were prepared in Chem3D 16.0 and AutoDock 4.2 (Python Molecule Viewer), then screened against computer generated models using AutoDock4.2 and the Racoon virtual screening tool. A Lamarckian Genetic Algorithm was selected for the simulation.

OBPs and ORs were all screened against a wide range of ligands. The binding energy of the complex and subsequent $K_i$ were calculated.

Molecular Dynamics

Molecular dynamics simulations were performed using GROMACS 2019. For odorant-binding proteins, the OLPS force field was used, and for odorant-receptors, a modified Gromas 53a6 force field was utilised.

For odorant-binding proteins, models were solvated and neutralised with ions (for negative $Cl^-$, for positive $Na^+$) before energy minimisation and equilibration (temperature and pressure) calculations were performed. For each protein, a 1 ns simulation was performed. The stability of the protein was then assessed by observing temperature and pressure stabilisation of the simulated models.

Molecular dynamics simulations were performed using the GROMACS 9.3 package. For OBPs, and OLPS force field was used, whereas for odorant-receptors, the Gromos53a6 forcefield was utilised, with modifications made to the Gromos53a6 forcefield parameters to include lipids and lipid topology. Lipid bilayer topology and structure were obtained from http://wcm.ucalgary.ca/tieleman/downloads. Receptor models were embedded into a lipid bilayer of 128 dipalmitoylphosphatidylcholine (DPPC) molecules. All models were solvated with explicit solvent (SPC) water and neutralised with either $Cl^-$ or $Na^+$ ions. Steepest-descent methods were used to minimise the energy of each system, at a reference temperature of 300 K. All bonds were constrained with LINCS and long-range electrostatics were handled using PME.

**Table 2:** Aphid sex pheromone components and analogues were screened against wild type *Acyrthosiphon pisum* OBP6.

| Example | Compound | Binding energy (kcal $mol^{-1}$) | $K_i$ (μM) |
|---|---|---|---|
| Example 1 (reference) | 1 | -8.76 | 0.38 |
| Example 2 (reference) | 2 | -7.75 | 2.08 |
| Example 3 (reference) | 3 | -7.77 | 2.01 |
| Example 4 (reference) | 7 | -7.87 | 1.69 |
| Example 5 (reference) | 8 | -8.15 | 1.06 |
| Example 6 (reference) | 10 | -7.78 | 1.99 |
| Example 7 (reference) | 12 | -8.05 | 1.26 |
| Example 8 (reference) | 13 | -8.29 | 0.83 |
| Example 9 (reference) | 16 | -8.45 | 0.64 |
| Example 10 (reference) | 17 | -8.04 | 1.27 |
| Example 11 (reference) | 19 | -8.39 | 0.71 |

(continued)

| Example | Compound | Binding energy (kcal $mol^{-1}$) | $K_i$ (μM) |
|---|---|---|---|
| Example 12 (reference) | 24 | -8.24 | 0.91 |
| Example 13 (reference) | 26 | -7.84 | 1.79 |
| Example 14 (reference) | 27 | -7.88 | 1.65 |
| Example 15 (reference) | 28 | -8.02 | 1.31 |
| Example 16 (reference) | 29 | -8.24 | 0.91 |
| Example 17 (reference) | 30 | -7.86 | 1.71 |
| Example 18 (reference) | 31 | -8.10 | 1.14 |
| Example 19 (reference) | 32 | -8.12 | 1.10 |
| Example 20 (reference) | 33 | -7.77 | 2.01 |
| Example 21 (reference) | 34 | -7.91 | 1.57 |
| Example 22 (reference) | 35 | -8.14 | 1.07 |
| Comparative Example 1 | A | -7.67 | 2.37 |
| Comparative Example 2 | B | -7.52 | 3.07 |
| Comparative Example 3 | C | -7.69 | 2.30 |
| Comparative Example 4 | D | -7.60 | 2.69 |
| Comparative Example 5 | E | -7.00 | 7.38 |

Structures of Compounds 1, 2, 3, 7, 8, 10, 12, 13, 16, 17, 19, 24 and 26-35, Compound A ((1*R*,4a*S*,7*S*,7a*R*)-nepetalactol), Compound B ((4a*S*,7*S*,7a*R*)-nepetalactone), Compound C ((1*S*,4a*R*,7*R*,7a*S*)-nepetalactol), Compound D ((4a*R*,7*R*,7a*S*)-nepetalactone) and Compound E ((1*R*,2*S*,5*S*)-dolichodial) are provided below.

Structures of Compounds 1, 2, 3, 7, 8, 10, 12, 13, 16, 17, 19, 24 and 26-35 and Compounds A-E:

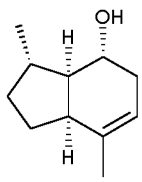

**1**

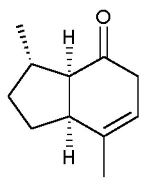

**2**

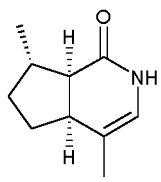

**3**

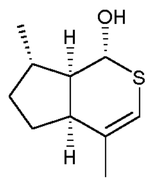

**7**

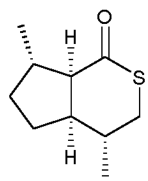

**8**

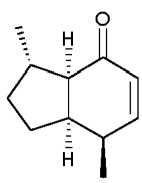

**10**

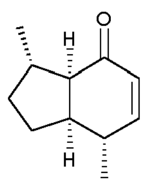

**12**

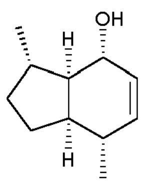

**13**

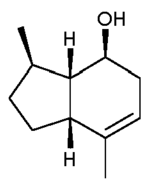

**16**

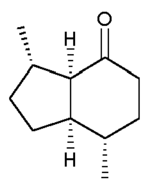

**17**

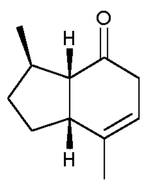

**19**

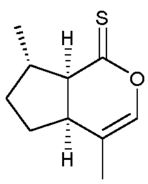

**24**

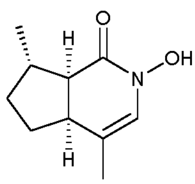

**26**

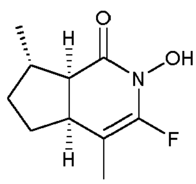

**27**

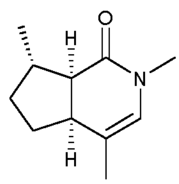

**28**

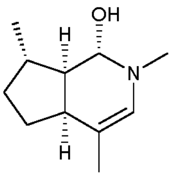
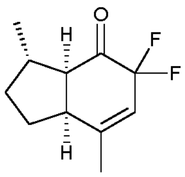
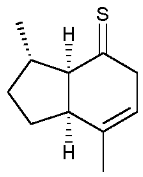
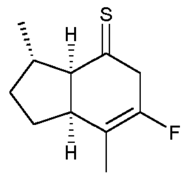
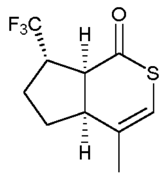

29 30 31 32 33

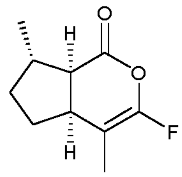
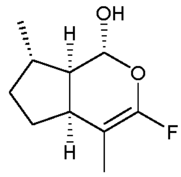

34 35

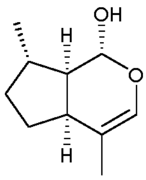
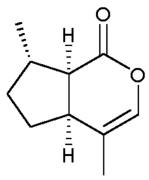
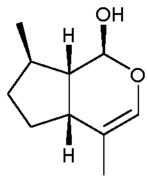
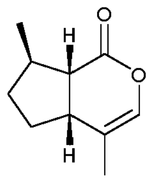
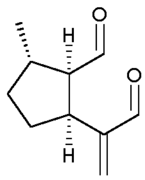

A B C D E

Referring to Table 2, the compounds of Examples 1 to 22 (reference) have a strong binding affinity to wild type *Acyrthosiphon pisum* OBP6. The compounds of Examples 1 to 22 also have high binding affinity compared with the compounds of Comparative Examples 1 to 5.

Figure 5(i) shows that the lone pair on the oxygen atom within the six-membered ring of natural nepetalactone is held close to another oxygen lone pair on Tyr176. Without wishing to be bound by theory, it is postulated that a change from an oxygen atom to a carbon, nitrogen or sulfur atom (e.g. Examples 1-11 and 13-20 (reference)) helps to increase binding affinity as there is less electrostatic repulsion (in the case of carbon and nitrogen, the lone pair is removed; in the case of sulfur, the lone pair is more diffuse with a less electronegative sulfur atom). It is also postulated that in the case of sulfur, the larger size of the sulfur atom leads to puckering of the ring system away from the oxygen lone pair on Tyr176, further enhancing binding affinity. Other structural changes (e.g. in Examples 12, 21 and 22) also help to increase binding affinity.

**Example 23: Docking of Aphid Sex Pheromones with Wild Type *Acyrthosiphon pisum* OBPs and ORs**

**Table 3:** Aphid sex pheromone components were screened against wild type *Acyrthosiphon pisum* OBPs.

| Ligand | Binding energy (kcal mol$^{-1}$) of OBPs | | | | | | | | | | |
|---|---|---|---|---|---|---|---|---|---|---|---|
| | 1 | 2 | 3 | 4 | 5 | 6 | 7 | 8 | 9 | 10 | 11 |
| A | -5.85 | 8.79 | -6.14 | -5.94 | -4.28 | -7.67 | -5.99 | -5.89 | -5.31 | 1.26 | -5.92 |
| B | -6.60 | 2.33 | NA | -5.86 | -5.00 | -7.52 | -6.03 | -6.15 | -5.79 | 2.60 | -6.44 |
| C | -6.05 | 10.78 | NA | -6.45 | -4.24 | -7.69 | -6.00 | -6.38 | -5.47 | 10.06 | -5.94 |
| D | -6.53 | 10.1 | NA | -6.17 | -5.07 | -7.60 | -5.98 | -6.33 | -5.71 | NA | -6.39 |

(NA = no favourable docking conformations were found in the screening)

**Table 4:** Aphid sex pheromone components were screened against wild type *Acyrthosiphon pisum* ORs.

| Ligand | Binding energy (kcal mol$^{-1}$) of ORs | | | | | |
|---|---|---|---|---|---|---|
| | 2 | 10 | 17 | 20 | 22c | 39 |
| A | -6.8 | -6.31 | -7.19 | -7.25 | -6.82 | -6.32 |

(continued)

| Ligand | Binding energy (kcal $mol^{-1}$) of ORs | | | | | |
|---|---|---|---|---|---|---|
| | 2 | 10 | 17 | 20 | 22c | 39 |
| B | -6.8 | -7.02 | -7.2 | -7.28 | -6.85 | -6.72 |
| C | -6.83 | -6.68 | -7.38 | -7.19 | -6.52 | -6.66 |
| D | -6.84 | -6.75 | -7.26 | -7.39 | -6.81 | -6.68 |

Referring to Table 3, OBP6 may have strong binding affinity to the aphid sex pheromone components, for both the biologically active enantiomers and the inactive enantiomers.

**Example 24: Expression and Purification of OBPs**

Media Recipes

Media for use in molecular biology experiments were prepared as in Table 5.

**Table 5:** Media recipes for LB, 2xYT and LB agar.

| Media | Components (+ 1L $H_2O$) |
|---|---|
| LB liquid media | 8g Tryptone, 5g Yeast Extract, 5g NaCl |
| 2xYT liquid media | 16g Tryptone, 10g Yeast Extract, 5g NaCl |
| LB agar | 30g LB agar mix (without NaCl), 5g NaCl |

Media was sterilised by autoclaving at 120°C, then cooled before adding an appropriate antibiotic to a working concentration (Table 6). Agar was kept at 60°C until poured into plates and allowed to set.

**Table 6:** Working concentrations of antibiotics added to media.

| Antibiotic | Working Concentration / $\mu g\ ml^{-1}$ |
|---|---|
| Ampicillin | 100 |
| Kanamycin | 50 |

Transformation of E. *coli* BL21(DE3) Competent Cells with *A. pisum* OBP Plasmids

All genes were previously cloned into a vector plasmid, pET45b or pET15b, containing an ampicillin resistance cassette, or pNIC28-Bsa4, containing a kanamycin resistance cassette. Each plasmid contained a hexa-histidine ($His_6$) tag encoded at the *N*-terminus of the protein, to allow for nickel affinity purification.

To transform cells, 5 $\mu$L of plasmid was added to BL21(DE3) competent *E. coli* cells and cooled to 0°C. The mixture was allowed to sit for 30 minutes, after which it was heat-shocked at 42°C for 70 sec and cooled to 0°C for a further 5 minutes. LB liquid media (800 $\mu$L) was added to the sample, followed by incubation (250 rpm; 37°C) for 45 minutes. The sample was centrifuged (4000 rpm) for 2 minutes and the pellet resuspended in LB media (100 $\mu$L). The resuspension was spread onto an LB-agar plate containing the appropriate antibiotic and incubated (37°C) for 12 hours. A clonal cell template for PCR was made from the overnight culture by diluting a scraping from a small, single colony in $H_2O$ (10 $\mu$L).

Polymerase Chain Reaction (PCR)

A mixture for PCR was made by combining the cell template (1 $\mu$L), appropriate forward and reverse primers (1 $\mu$L of each), $H_2O$ (7 $\mu$L) and a Taq polymerase mix (10 $\mu$L) containing dNTP, Taq polymerase and PCR buffer (100 mM Tris-HCl, 500 mM KCl, 15 mM $MgCl_2$, pH 8.3). The mixture was heated at 94°C for 5 minutes, followed by 25 cycles of heating at 94°C for 30 seconds, to 50°C for 30 seconds, then 72°C. Finally, the mixture was taken to 72°C for 30 minutes and stored at 4°C. Gel electrophoresis with a 1% agarose gel was used to check the PCR product and determine whether the transformation was successful.

Recombinant *E. coli* BL21(DE3) Starter Culture

A scraping from clonal culture or glycerol stock sample (500 μL) was added to 2xYT liquid media (10 mL) and incubated overnight (37°C; 250 rpm) to generate a starter culture of *E. coli* BL21(DE3).

Protein Expression Test from Recombinant BL21(DE3) *E. coli*

Starter culture of recombinant BL21(DE3) *E. coli* cells (500 μL) was added to 2xYT liquid media containing the appropriate antibiotic (10 mL) and incubated (37°C; 250 rpm) until the $OD_{600}$ values reached 0.5-0.6. Isopropyl β-D-1-thiogalactopyranoside (IPTG; 10 μL; 1M) was added to induce expression, and the mixture incubated for a further 3-4 hours (37°C; 250 rpm).

A sample of the culture (2 mL) was removed and centrifuged (12000rpm; RT) for 2 min, and the cell pellet resuspended in SDS-PAGE loading buffer (600μL). Samples were then denatured at 95°C for 10 minutes, before loading onto an SDS-PAGE gel.

Large Scale Expression and Refolding of OBPs

2xYT liquid media (600 mL) was inoculated with starter culture (3 mL) and incubated (37°C, 250 rpm) until an $OD_{600}$ of 0.7-0.8 was reached. IPTG (300 μL; 1 M) was added to induce expression and the mixture incubated for a further 3-4 hours (37°C, 250 rpm). Cells were harvested by centrifuging for 15 minutes (3500 rpm).

The cell pellet was resuspended in ice-cold TBS buffer (10 mL; 25 mM Tris, 500mM NaCl, pH 8.0) and incubated on ice for 10 minutes. The mixture was sonicated for 4 minutes on ice, then centrifuged for 30 minutes (35000 rpm, 4°C). The pellet was resuspended in TBS+0.2% Triton X-100 (10 mL). The sonication and centrifugation steps were repeated, and the pellet resuspended in 8M urea (1.5 mL) and 10 mM 1,4-dithiothreitol in 100 mM Tris (1.5 mL; pH 8). The mixture was incubated for 1 hour at room temperature, then rapidly diluted with 27 mL TBS+5:0.5 mM GSH:GSSG. The diluted mix was incubated overnight at RT.

The overnight mixture was centrifuged for 10 minutes (3500 rpm) and the supernatant filtered through a 0.22 μm filter.

Purification of OBPs

OBPs were purified using Histrap® columns, preconditioned with 25 mM imidazole buffer. The filtrate from the refolding step (Chapter 3; 3.2.5) was passed through the column, leaving the histidine-tagged protein bound to the nickel. An imidazole buffer (500 mM) was used to displace the protein and fractions were collected and analysed using SDS-PAGE.

Sodium Dodecyl Sulfate Polyacrylamide Gel Electrophoresis (SDS-PAGE)

The resolving gel (1.0 mm width; 5 mL) was prepared as in Table 7. Approximately 1.5 mL of 2-isopronaol was added to remove bubbles and the gel left to set. After 20 minutes, the isopropanol was removed, and the stacking gel added. A comb was used to form wells before leaving the gel to set for a further 20 minutes.

**Table 7:** Composition of resolving and stacking gels for SDS-PAGE.

| | **Volume / μL** | |
|---|---|---|
| | **Resolving gel (15%; 5 mL)** | **Stacking Gel (3%; 2mL)** |
| $H_2O$ | 1770 | 1185 |
| 40% acrylamide | 1875 | 150 |
| 1.5M Tris buffer (pH 8.8) | 1250 | 0 |
| 0.5M Tris buffer (pH 6.8) | 0 | 250 |
| 10% SDS buffer | 50 | 25 |
| 10% ammonium persulfate* | 50 | 25 |
| TEMED* | 5 | 3 |
| (*added last to catalyse polymerisation) | | |

Samples for the gel (20 μL) were prepared by combining protein samples (10 μL) with sample buffer (10 μL;

prepared as in Table 8). Samples were run alongside an appropriate marker (11-245 kDa). The gel was run at 200 V for 90-120 minutes and GelCode™ Blue Stain (Thermo-Fisher Scientific) was added for approximately 1 hour to resolve the protein bands.

Table 8: Composition of SDS-PAGE Protein Sample Buffer

| Reagent | Concentration |
|---|---|
| Tris | 80 mM |
| SDS | 2.0% |
| Glycerol | 10% |
| Bromophenol blue | 0.0006% |
| DTT | 0.1 M |

Histidine-Tag Cleavage from Purified Protein

To remove the hexa-histidine tag from purified proteins, an appropriate cleavage enzyme was used (Table 9). The selection of the enzyme depended on the cleavage site encoded in the original vector plasmid. The protein was buffer-exchanged in $CaCl_2$ in TBS buffer (2 mM $CaCl_2$, 25 mM Tris; 500 mM NaCl), and enzyme added. Cleavage was monitored by observing a change in mass using mass spectrometry. After completion, excess tagged protein was removed by passing the mixture through a Histrap® column and collecting the eluent.

**Table 9:** Vector plasmids used for transformation and their His-tag cleavage sites and enzymes.

| Plasmid | His-Tag FASTA sequence | Cleavage Site | Cleavage Enzyme |
|---|---|---|---|
| pET45b | MAHHHHHHVG TGSNDDDDKS PDP (SEQ ID NO: 30) | DDDDK/S (SEQ ID NO: 33) | Enterokinase |
| pNIC28-Bsa4 | MHHHHHHSSGVDLGTENLYFQS (SEQ ID NO: 31) | ENLYFQ/S (SEQ ID NO: 34) | TEV (Tomato Etch Virus) |
| pET 15b | MGSSHHHHHHSSGLVPRGSH (SEQ ID NO: 32) | GLVPR/GS (SEQ ID NO: 35) | Thrombin |

Plasmid Extraction, Purification and Sequencing

Plasmids were extracted using a GeneJET Plasmid Miniprep Kit (Thermo-Fisher Scientific) and sequenced by Queen's Medical Centre laboratories, University of Nottingham or Eurofins (UK).

Protein Buffer Exchange and Concentration

All proteins were buffer exchanged and desalted using a PD-10 desalting column and concentrated using a Vivaspin 20 (10 kDa MWCO).

Site-Directed Mutagenesis

Site-directed mutagenesis (SDM) of OBP6-$His_6$ was performed to insert a thrombin tag. A Q5 Site-Directed Mutagenesis kit (New England Biolabs) was used to perform the mutagenesis. Primers (Table 10) were obtained from Sigma-Aldrich.

**Table 10:** Forward and reverse primers for site-directed mutagenesis (thrombin cleavage site insertion) of OBP6-$His_6$

| Primers for thrombin cleavage site insertion | |
|---|---|
| Forward | cgcggcagcGCTGGGTACGATAGAACATG |
| Reverse | cggcaccagCGGATCCGGACTCTTGTC |

Samples for PCR were prepared by combining plasmid containing the gene of interest (1 μL), forward and reverse primers (1 μL of each), $H_2O$ (9 μL) and Q5 hot start master mix (12.5 μL).The mixture was heated at 98°C for 30 seconds, followed by 25 cycles of heating at 98°C for 10 seconds, to 64°C for 20 seconds, then 72°C for 2 minutes. Finally,

the mixture was taken to 72°C for 2 minutes and stored at 4°C.

The PCR product (1 μL) was mixed with KLD reaction buffer (5 μL), KLD enzyme mix (1 μL) and $H_2O$ (3 μL) and left at RT for 5 minutes. Finally, the mixture (5 μL) was used to transform NEB 5-alpha Competent *E. coli* cells as described under "Transformation of E. coli BL21(DE3) Competent Cells with A. pisum OBP Plasmids". Results were validated by sequences as described under "Plasmid Extraction, Purification and Sequencing".

Fast-Protein Liquid Chromatography

To purify proteins, gel filtration *via* fast-protein liquid chromatography (FPLC) was performed using an Akta FPLC. The FPLC was fitted with a Superdex size-exclusion column, and samples were exchanged into a TBS buffer (Tris 15 mM, NaCl 250 mM; Table 11). Protein samples were collected using an autosampler and subsequently concentrated as described under "Protein Buffer Exchange and Concentration".

**Table 11:** Run conditions for size exclusion fast-protein liquid chromatography (FPLC).

| Step | Time | Flow Rate | Buffers |
|---|---|---|---|
| Wash | 20 min | 3.0 mL $min^{-1}$ | 1:120% EtOH:$H_2O$ |
| Wash | 20 min | 3.0 mL $min^{-1}$ | 1:1 TBS:$H_2O$ |
| Equilibrate | 40 min | 1.5 mL $min^{-1}$ | TBS |
| **Inject Sample** | | | |
| Run | 60-180 min | 1.5 mL $min^{-1}$ | TBS |
| Wash & Store | 40 min | 3.0 mL $min^{-1}$ | 20% EtOH |

Mass Spectrometry Analysis

To confirm efficient cleavage of the His-tag and determination of the structure, denatured mass-spectrometry of the recombinant proteins was performed using a Waters QTof2 spectrometer. Samples were prepared using a ZipTip® before injected into the mass spectrometer.

General Mass Spectrometry

All mass spectrometry was undertaken using electrospray ionisation and conducted on the QTof2 or QTof3 under denaturing conditions (80% $CH_3CN$, 0.1% formic acid) and the settings detailed in Table 12, unless otherwise stated.

Mass spectra were analysed using MassLynx 3.0 software.

**Table 12:** Denatured mass-spectrometry settings for the QTof 3.0

| Setting | Value |
|---|---|
| Capillary Voltage | 2.80 kV |
| Cone Voltage | 35 V |
| Desolvation Temperature | 80°C |
| Source Temperature | 50°C |
| Rf Lens | 1.0 |

Sample Preparation using the ZipTip®

Samples for mass-spectrometry were prepared by ZipTip®. The ZipTip® was conditioned by washing with elution buffer (80% $CH_3CN$, 0.1% formic acid; 5x10 μL), followed by equilibrating with wash buffer (5% MeOH, 0.1% trifluoroacetic acid; 7x10 μL). Trifluoroacetic acid (TFA) was added to the sample to a concentration of 0.1%, which was adsorbed onto the column (20 aspirations). The sample was then washed with wash buffer (20x10 μL) and eluted into elution buffer (10 μL, 20 aspirations).

**Example 25: Fluorescence Based Assays of *A. pisum* Odorant-Binding Proteins and Interactions with Ligands**

Fluorescence Measurements

All fluorescent measurements were undertaken using a Perkin-Elmer LS50B fluorescence spectrophotometer, using a 2mL quartz cuvette, and the settings described below in Table 13, unless otherwise stated. Spectra were recorded using FL WinLab software.

**Table 13:** Settings for fluorescence measurements using the Perkin-Elmer LS50B fluorescence spectrophotometer

| Setting | Intrinsic Fluorescence (Tryptophan) | Probe Fluorescence (1-NPN) |
|---|---|---|
| Excitation Wavelength | 280 nm | 337 nm |
| Emission Wavelengths | 290 - 400 nm | 350 - 600 nm |
| Excitation Slit | 5.0 nm | 5.0 nm |
| Emission Slit | 5.0 nm | 5.0 nm |

Ligand Binding and Saturation Curves

Saturation of OBPs with fluorescent probe and 1-NPN (Sigma-Aldrich) was initially measured by titrating a 2 μM protein sample (2 mL in 25 mM Tris-HCl) with aliquots of 1 mM ligand (Sigma-Aldrich) in methanol to final concentrations of 1-16 μM. The fluorescence intensity at 330 nm was recorded.

The competitive binding of ligands was measured by observing the intrinsic fluorescence of tryptophan. Titrations were performed with aliquots of 1 mM ligand in methanol to final concentrations of 1-20 μM, either after the addition of fluorescent probe to a final concentration of 1 μM or in the absence of fluorescent probe.

**Table 14:** The calculated $K_D$ values for OBP6 and various ligands from different binding assays.

| Ligand | OBP6 | | OBP9 |
|---|---|---|---|
| | 1-NPN Assay $K_D$ / μM | Fluorescent Probe-Free Assay $K_D$ / μM | 1-NPN Assay $K_D$ / μM |
| (1*R*,4a*S*,7*S*,7a*R*)-nepetalactol | 2.62 ± 0.63 | 12.74 ± 2.31 | 5.74 ± 1.71 |
| (4a*S*,7*S*,7a*R*)-nepetalactone | 1.30 ± 0.60 | 1.90 ± 0.35 | 6.49 ± 1.58 |
| (1*S*,4a*R*,7*R*,7a*S*)-nepetalactol | 2.65 ± 0.80 | 8.46 ± 1.62 | 6.29 ± 1.99 |
| (4a*R*,7*R*,7a*S*)-nepetalactone | 4.37 ± 0.81 | 12.01 ± 4.24 | 9.68 ± 4.57 |
| (*E*)-β-farnesene | 10.12 ± 2.88 | 34.47 ± 10.85 | 8.32 ± 2.67 |
| (*R*/*S*)-linalool | 8.95 ± 3.71 | NA | NA |
| (NA = not available or not measured) | | | |

Analysis of Fluorescence data

To generate $K_D$ values, relative fluorescence intensity was plotted against the concentration of ligand as a binding curve. $K_D$ values were generated in GraphPad Prism 7 using a non-linear regression and the inbuilt equation:

$$y = \frac{B_{max} * x}{K_D + x}$$

Each calculated $K_D$ value had an associated error from the non-linear regression. To account for these errors in statistical analysis, values were weighted in direct proportion to their error - the higher the error, the lower the weighting of the values. The 'weight' factor was calculated using the following equation:

$$weight = \frac{1}{(SE)^2}$$

Statistical analysis of fluorescence data was performed using R 3.4.4. A one-way weighted ANOVA was performed between ligands for each protein, and a two-way weighted ANOVA was performed to investigate the interactions between proteins and ligands. A Tukey Test was used for post-hoc analysis (Table 15).

**Table 15:** Statistical analysis results for a weighted analysis of variance (ANOVA) of the binding affinities ($K_D$) difference between different ligands and OBP6.

| **Assay** | **Interaction** | | ***p*-value** |
|---|---|---|---|
| | **Ligand A** | **Ligand B** | |
| 1-NPN | (1*R*,4a*S*,7*S*,7a*R*)-Nepetalactol | (*E*)-β-Farnesene | 0.0021 |
| | (4a*S*,7*S*,7a*R*)-Nepetalactone | | 0.00017 |
| (1*S*,4a*R*,7*R*,7a*S*)-Nepetalactol | | 0.010 | |
| (4a*R*,7*R*,7a*S*)-Nepetalactone | | 0.015 | |
| (1*R*,4a*S*,7*S*,7a*R*)-Nepetalactol | (±)-Linalool | 0.023 | |
| (4a*S*,7*S*,7a*R*)-Nepetalactone | | 0.0014 | |

The interaction between (4a*S*,7*S*,7a*R*)-nepetalactone and (4a*R*,7*R*,7a*S*)-nepetalactone did not give a significant difference, with a *p*-value of 0.11 in the 1-NPN assay and a *p*-value of 0.055 in the fluorescent probe free assay. A weighted t-test was subsequently performed, giving a significant difference with a *p*-value of $2.27 \times 10^{-4}$.

**Example 26: Saturation Transfer Difference NMR Experiments**

General NMR spectroscopy

Samples were run using an AVANCE Bruker DRX-500 MHz Nuclear Magnetic Resonance spectrometer with a 5 mm BBO BB-1H probe and set at 500 MHz for $^1$H spectra and 125 MHz for $^{13}$C spectra. Analysis of Bruker data was performed using Topspin 4.0.7, and analysis of Varien data performed with CCPNMR V2 and NMRPipe. Standard NMR tubes were used with a sample volume of 600 μL unless otherwise stated. Deuterated chloroform ($CDCl_3$), methanol ($CD_3OD$) and dimethyl sulfoxide (*d*-DMSO) were stored over 4 Å molecular sieves and used as both sample solvents and internal standards. For assignment of small molecules, additional 2D-NMR spectroscopy experiments were performed.

Standard sample preparation

Protein samples for NMR were desalted and buffer exchanged into 9:1 $H_2O$:$D_2O$ unless stated otherwise (as in "Protein Buffer Exchange and Concentration"). Ligand samples were not soluble in $D_2O$ and samples were prepared in d-DMSO. This resulted in a final NMR solvent including $H_2O$, $D_2O$ and *d*-DMSO.

Saturation Transfer Difference (STD) NMR

An initial test assay containing Bovine Serum Albumin (BSA) protein with tryptophan and sucrose was prepared for STD NMR. Samples were run for 192 scans, and the saturation transfer difference (STD) spectra generated.

**Table 18:** Composition of the final STD assays, both the BSA test assay and OBP6 assay.

| **Component** | **Test Assay** | **OBP6 Assay** |
|---|---|---|
| Protein (Unlabelled) | BSA; 100 μM | OBP6; 30 uM |
| Suspected Binder | Tryptophan; 10 mM | (4aS,7S,7aR)-nepetalactone); 3 mM |
| Suspected Non-Binder | Sucrose; 10 mM | Not Included |

STD absolute values were calculated by observing the change in proportions between the off-resonance spectrum and the final STD spectrum using the following equation:

$$STD\ absolute\ value = \frac{I_0 - I_{STD}}{I_0}$$

in which the term ($I_0$ - $I_{STD}$) represents the ratio of peak intensity in the STD spectrum and $I_0$ the ratio of intensity in the off resonance spectrum. A second value representing the proportionate change was calculated using the following equation:

$$Difference\ in\ proportion = I_0 - (I_0 - I_{STD})$$

Finally, epitope mapping was performed by calculating the relative peak integration in the STD spectrum vs the off-resonance spectrum of a peak as a percentage.

**Synthesis Examples 1-4 (reference)**

Nepetalactone and derivatives may be synthesized using (inverse electron demand) Diels-Alder reactions as described in Dawson et al. (Bioorganic Med. Chem. 1996, 4 (3), 351-361) and Schreiber et al. (J. Am. Chem. Soc. 1986, 108, 8274-8277). Some further modifications are illustrated below.

**Synthesis Example 1 (reference): (4a*S*,7*S*,7a*R*)-4,7-dimethyl-2,4a,5,6,7,7a-hexahydro-1*H*-cyclopenta[*c*]pyridin-1-one (Compound 3)**

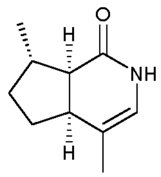

**3**

Ammonia gas was bubbled gently through a solution of nepetalactone (1.02 g, 6.12 mmol) in DCM (70 ml) for 1 hour. The reaction flask was then sealed and stirred for a further 16 hours. The crude reaction mixture was concentrated under vacuum and purified via distillation (Kugelrohr, 46 mbar, 162 °C) to yield the product. $\delta_H$ (500MHz, $CDCl_3$) 6.73 (1H, s), 5.62 - 5.70 (1H, m), 2.71 (1H, q, J = 8.4 Hz), 2.27 - 2.37 (2H, m), 1.98 - 2.08 (1H, m), 1.78 - 1.87 (2H, m), 1.64 (3H, s), 1.45 - 1.54 (1H, m), 1.21 (3H, d, J = 6.6 Hz).

**Synthesis Example 2 (reference): (4a*S*,7*S*,7a*R*)-4,7-dimethyl-5,6,7,7a-tetrahydrocyclopenta[c]thiopyran-1(4a-*H*)-one (Compound 23)**

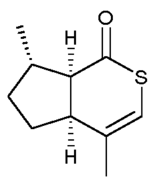

**23**

In order, indium triflate (0.17g, 0.30 mmol), toluene (7.5 ml), nepetalactone (1.00 g, 6.02 mmol), sulphur (0.21 g, 6.62 mmol) and phenyl silane (0.43 g, 4.01 mmol), under $N_2$, was added to a screw topped flask, sealed and heated to 120 °C for 24 hours. The reaction mixture was cooled and the pressure released carefully. The reaction mixture was filtered

through celite before being purified on silica gel (2% EtOAc in hexanes) to give the product (0.12 g, 12% yield) as yellow/brown oil. $\delta_H$ (500MHz, $CDCl_3$) 5.72 (1H, s), 2.84 - 2.94 (1H, m), 2.55 (1H, hept, J = 7.0 Hz), 2.38 (1H, t, J = 7.5 Hz), 1.89 - 2.00 (2H, m), 1.87 (3H, s), 1.69 - 1.79 (1H, m), 1.17 - 1.32 (1H, m), 1.01 (3H, d, J = 6.8 Hz).

**Synthesis Example 3 (reference): (4a*S*,7*S*,7a*R*)-4,7-dimethyl-5,6,7,7a-tetrahydrocyclopenta[*c*]pyran-1(4a*H*)-thione - nepetathionolactone (Compound 24)**

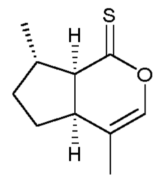

**24**

To a solution of nepetalactone (2.00 g, 12.03 mmol) in acetonitrile (30 ml) was added phosphorus pentasulphide (2.68 g, 6.02 mmol) followed by hexamethyldisiloxane (4.92 g, 30.30 mmol) and heated to 82 °C for 16 hours. The solvent was removed under vacuum and the crude product purified on silica gel (2% EtOAc in pet ether) to give the product (0.42 g, 37% yield) as a yellow oil. $\delta_H$ (500MHz, $CDCl_3$) 6.37 (1H, s), 2.95 (1H, t, J = 8.5 Hz), 2.70 - 2.77 (1H, m), 2.28 (1H, hept, J = 7.4 Hz), 1.81 - 1.92 (2H, m), 1.72 - 1.81 (1H, m), 1.62 (3H, s), 1.28 - 1.33 (1H, m), 1.19 (3H, d, J = 6.7 Hz).

**Synthesis Example 4 (reference): (4a*S*,7*S*,7a*R*)-4,7-dimethyl-1,4a,5,6,7,7a-hexahydrocyclopenta[*c*]pyran-1-thiol (Compound 25)**

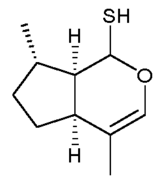

**25**

To a solution of nepetathionolactone (Compound 24) (0.35 g, 1.90 mmol) in isopropyl alcohol (45 ml) was added sodium borohydride (0.10 g, 2.66 mmol) and the mixture stirred for 2 hours. The reaction was quenched with 12 ml of 2M HCl and saturated brine was added. The aqueous layer was extracted with diethyl ether. The combined organic layer was dried ($MgSO_4$) and concentrated under vacuum. The crude product was purified on silica gel (15% $Et_2O$ in pet ether) to give the product (0.21 g, 60% yield) as a pale yellow oil. $\delta_H$ (500MHz, $CDCl_3$) 6.00 (1H, s), 4.23 (1H, d, J = 4.3 Hz), 2.75 (1H, q, J = 7.7 Hz), 1.80 - 2.32 (4H, m), 1.43 - 1.57 (1H, m), 1.79 (3H, s), 1.29 - 1.38 (1H, m), 0.91 (3H, d, J = 6.7 Hz).

**Fabrication Example 1: Biosensor comprising ApisOBP6**

A sensing surface (a film bulk acoustic wave resonator - FBAR - fabricated by Sorex Sensors) was functionalised via the APTES-glutaraldehyde method. The sensing surface to be functionalised was exposed to a stream of ozone for 15 mins, before being allowed to stand in air for 30 mins and washed with distilled water. To the freshly oxidised sensing surface, a solution of APTES (2% in ethanol) was added and ensured the whole sensing surface was covered and allowed to stand for 10 mins. The excess reagent was removed with clean ethanol, before being dried under a stream of nitrogen and cured in an oven (110 °C) for 60 mins. After cooling to RT, a 1% solution of glutaraldehyde was added to the surface for a further 60 mins. A solution of the desired OBP (ApisOBP6, 1 μM) was added to the surface for 1 hour. The functionalised sensing surface was washed with distilled water and stored until required.

While the foregoing disclosure provides a general description of the subject matter encompassed within the scope of the present invention, including methods, as well as the best mode thereof, of making and using this invention, the following examples are provided to further enable those skilled in the art to practice this invention and to provide a complete written description thereof. However, those skilled in the art will appreciate that the specifics of these examples should not

be read as limiting on the invention, the scope of which should be apprehended from the claims and equivalents thereof appended to this disclosure. Various further aspects and embodiments of the present invention will be apparent to those skilled in the art in view of the present disclosure.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

## SEQUENCE LISTING

**SEQ ID NO: 1** (OBP1 from *Acyrthosiphon pisum)*

MLNLKVMMFLCLSVIVVYCESDQVPINSSAAVESCLLETNMTRDEFEDMLTSPNARELTILKSH
AHKCMFGCVMRKNHIVNDGVVSKEVLSKYVLNFYGRPDYKRRLIIKDVEHIVDVCAKKVADESE
TDECELAATLVTCIVLEANKAGLVDDPARQI

**SEQ ID NO: 2** (OBP2 from *Acyrthosiphon pisum)*

MKVSAATAVLVALVATVQSSDPCNISTCYKSGTTKPPMAVTPTHLPVQSSSTQTSHPQTTYAKD
HVHGSTTTKSGVNATVTTASGASVNGTEPPAVVKSSAGVTGNSTTPKPTMTEGHVALKQKLNTI
AVKCKDELHAPQEIMALVSNTVVPQNEQQRCYLECVYKNLNLIKNNKFSVEDGKAMARIRFANQ
PEEHKKAVTIIETCEKEAVIDPKTTEKCAAGRVIRNCFVKNGEKINFFPKA

**SEQ ID NO: 3** (OBP3 from *Acyrthosiphon pisum)*

MISSTFYITLVFGIAMLISCGHGRFTTEQIDYYGKACNASEDDLVVVKSYKVPTTETGKCLMKC
MITKLGLLNDDGSYNKTGMEAGLKKYWSEWSTEKIESINNKCYEEALLVSKEVVATCNYSYTVM
ACLNKQLDLDKST

**SEQ ID NO: 4** (OBP4 from *Acyrthosiphon pisum)*

MRGNYSSMVFLLFAIGFQDIFCQKQEPSGKCRAPDKAPLNLEIIINTCQEEIKSALLQEALDIL
NDGNVEQNTPNYSSRSKREAEEDLTNEERRVAGCLLQCVYKKVKAVDETGFPVVDGLMKLYNEG
VQDRNYYIATLSAVRHCISIAQQLKQQQPSKSFDDGQTCDLAYEMFECVSEKIEENCGVENKSN
N

**SEQ ID NO: 5** (OBP5 from *Acyrthosiphon pisum)*

MSANSATIKCIAVAAILLQISVIFADAGHHRRGKELLDTEDSDFFRCKQASRKSCCGPENAMKR
FGDKDKVAADECYAQVAEKFATVTATTPKQDLFSAEAVKITKKKQFCLHECIGKKNNLLTEDGS
LNKTFIADYAMKSVFKEQWQKQVGQKALDKCLEETYIPWPAEDKENVCNPVYVQFQHCLWLQYE
SNCPANKIKITKKCEKTRNRYRMQKSTSN

**SEQ ID NO: 6** (OBP6 from *Acyrthosiphon pisum)*

MQKVVFICIFAIICQTVFTAGYDRTWILRQKRGTNDDECRTLLPSSEKKLPSCCQMPNILPNLD STWEKCFETFKQFKDKPETKEYKEMAHGKEPPCLFQCIFMQSGLTTSDGKLNEDAITKKMSEGI NNDEKWKSIWQNSLNKCFDDVKQEDKKQILIMNTPAGRLMKCFLRDMYMSCPKNVWVESSECLN MKDLVQKCPEMPPPVFKSPPKLI

**SEQ ID NO: 7** (OBP7 from *Acyrthosiphon pisum)*

MVARKRMYNMLPTTVLFAIIAATVLKDCDAYLSEAAIKKTQQMLKTVCSKKHSVEEDVFTNIKK GIFPEDNNNIKCYFACNFKTMQLINQKGVIDKKMFKDKMSMMAPPNVYKILLPVIEQCTGKDKG EELCQSSYNVIKCAHSVDPKSLEFLPL

**SEQ ID NO: 8** (OBP8 from *Acyrthosiphon pisum)*

MFALKVACLCLSVAVVFGENNQQNGPSDRSATIFQSCIAETKLSGDALKGFRSMSIPKTQAEKC MMGCLMRKVNVINKGKFSVEEATKVAQKYYGTNEAMMKKAKDLIDVCAKKAQSTTEECALAGIV TTCIVEEAQKAGLSGGPGSRSRRTVSPKFRRDAM

**SEQ ID NO: 9** (OBP9 from *Acyrthosiphon pisum)*

MIIKKTLLLSVFVLFGCLFSINKADDADAKDKELMSKLFTVVFKCFKDADWGTCGEMITTKYDI TQAKYKQCTCHMACAGEELGMINASGQPEPAKFLEYVNKINNPDIKSQLQLIYDKCQNVKGSEK CDLAEQFAICAFKESPALKERVSTLMEMLVKMKPKSK

**SEQ ID NO: 10** (OBP10 from *Acyrthosiphon pisum)*

MEHLRSTNVVFAIVMALLVVQSSTRPQPDEMEEIKRTLYNACAGKFPITEEIKNNAKNSIISDD PTFKCFLKCCFDEMSMIDEDGIIDGDSLKAMAPDHIKPILEQVIPSCTKNVKQDGCEASFEFIS CGIKLNPLIVALLPL

**SEQ ID NO: 11** (OBP 11 from *Acyrthosiphon pisum)*

MSSSTFYITLLFGIAMLISCGYGIFTTEQIDYYGKACNASEDDLIVLKSYKVPSTETGKCLMKC MITKLGLLNDDGSYNKTGMEAGLKKYWSEWATEKIETINEKCYEEGNTATLLYHVAIYFTCVSG DYSDVQLLIHCDGMFEQEVGSRQVNLKLLIMLKIGLSEPKR

**SEQ ID NO: 12** (ORCO from *Acyrthosiphon pisum)*

MGYKKDGLIKDLWPNIRLIQLSGLFISEYYDDYSGLAVLFRKIYSWITAIIIYSQFIFIVIFMV TKSNDSDQLAAGVVTTLFFTHSMIKFVYFSTGTKSFYRTLSCWNNTSPHPLFAESHSRFHAKSL SRMRQLLIIVSIVTIFTTISWTTITFFGESVWKVPDPETFNQTMYVPVPRLMLHSWYPWDSGHG LGYIVAFVLQFYWVFITLSHSNLMELLFSSFLVHACEQLQHLKEILNPLIELSATLDSSVHNPA EIFRANSAKNQSINGIDHDYNGSYVNEITEYGTKGENEPNRKGPNNLTSNQEVLVRSAIKYWVE RHKHVVKYVSLITECYGSALLFHMLVSTVILTILAYQATKINGVNVFAFSTIGYLMYSFAQIFM FCIHGNELIEESSSVMEAAYGCHWYDGSEEAKTFVQIVCQQCQKPLIVSGAKFFNVSLDLFASV LGAVVTYFMVLVQLK

**SEQ ID NO: 13** (OR2 from *Acyrthosiphon pisum)*

MDVMQKPERFILTPFQKFCIRWSVFFDSSSDRLSRIETVLRTIQFSTIMITSGMTMTSVLIADN
KKALESFTYFVICVFMLAIITFAIRTKRFNRAMLLMVVDEFPGYNRPMPDVLKRKMAAIRTSYG
DFTMKVIVSYLTLVLFEIPATAMVPLAAASLTDVKLGSQSTQMVVLWFPADTSQVGMYAVSYVI
QFLIVVTVKFIITGIMCSFSFFVSQMISEFQILSAYVEHAVEIVEYDQSADKTTEQKLLDHVKN
CVMLHDRLIYFKDQLNESYGYIILLELMFSTLYFCLSAFNMIFVGNRFVMVKGLLTLSNYLAEL
FIFCMYGSMVEDAHMGLLRASYSVAWYAQPVRFRQSLTMVMSRTQTPLQLTVGKVFIANLPLFL
SVLKVSYSGVNALRAANAK

**SEQ ID NO: 14** (OR4 from *Acyrthosiphon pisum)*

MTSIGKKNQRKFYQTLMTLAFFLDTSQYRYISRFVKQFYIFDWMVLVSVAAAFTILEGNYRMPF
VMELIQYMIVGFYFTSIFVVFIIKKEAIMSNYNCIQTKFIQWSNKRALHSNAAYKRNIKTVKSL
SIPLAILSLSIALGPLISTINDIGKLPLDNRAHFVLFWPTIVDTNKLSMYGIIYTLQVIFTIIL
YISVLSFNLGYMVFLNELITQFEMLLNGINDAFKYKMDKQFQTLFIDCIRHHQIIIKFLDDLKS
YFKWMILIEIIVVQVILAILIYNLTKVNASLGYKVKIAGSILFNLLPICFHCHVGEVVLSLHTR
LSNHIYNMPWYDMPNKNKQLIVIMLQRTQRDLTLSSALFSSERASRSLISKVIKQVYTILNVLL
KT

**SEQ ID NO: 15** (OR5 from *Acyrthosiphon pisum)*

MQRIDTINMFLQMTGCTDSKAMLYLTYFEFLITFYYLIATYASIVHFEQSVTIQLFALLCMLIE
CVILLNITFRLYHKNHIREMHQYSRRLGIPDSYRSVINVITKYHLIASNIFVVFPVTYAIFCDS
VRVGDPFTFPFLDVLPMHTDNLAIYACKYLVYAISVYIAHVELCFINTTFIYYVGVLKHRLETI
VQTIGEAFADNDEQKFKYAIIQHQKLLSYFNTMKIVFSKPILLSMSFNAIYFGLTTSFVIQAIR
GYINQAILSICIASSAAAVINITIYTFYGSELMDLHDKILHVLFDNAFFYVSKSFKSSILIMMT
RVTIPLKFTVGYIFTINLNLLLKILKMSYTVLNVLLSSETIKPHKLS

**SEQ ID NO: 16** (OR10 from *Acyrthosiphon pisum)*

MAHIVDIFFQNMGCSHDHGYGMVFFNCCELAITLFFTVSTYPTIADPTQNLSIRLYGVLCLLIE
AHIFAFIAVRIYHQSQHRDMYQHLHGVEIPENYRRKIATVIKHHFIISNVFVAVSVLYTISLDW
VRIGDPFTFPFIDVLPIKTTNVTVYVCKYIVYALPVYFAHLETCFLNVTFMFSVGIVKRHFQIL
NDQVEEAIVNEDEQKLKIAIKHHQQVLKYFEDMKTVYEKPILMTIEFCGLYVGLTSCFVIQVIQ
GFIHQIILGLCIVSSIACLMTIIIYCIYASNMYALHNGILNALFEHRSCYSRNKSFKRIILIMM
TRATIPLEIKAGSVFTINLNLLVKILKFAYTVFNVLLSSINRQFKETAI

**SEQ ID NO: 17** (OR17 from *Acyrthosiphon pisum)*

MTTTPRVTELTAPASEDLTIVDNRLFKAICLHQILDPTKGGNRYYRLAFMVVMWVSLSVQIIQL
VGLYFAVNDLQRFAFTTTVIFNALLCLSKGYVLVVNADRLRASLEVARYEFTSCGARNQRLVRR
SRAVLSTILRTFAVLSWVTCFIWALTPLFAMDEYLQVTNADGTVSRYRVTIYNVWLPVPATVYN
ETTVWSLVYAVEVIACFVNVFSWLLFDSYVVTMCFTFNAQFRTVSASTTIGHHSDSFRSPPPHA
PEGTSDDNNTFNCYDELINRIKDNQSIIKIYDDFFEILQPAILFQIIGGSYSVITLIFLTSLTY
LMGFSIISIPVLKVFFGFLSVTFELFLYCYVFNHIETEKCNMNFGLYSSNWTAMDLKFKKTLLF
AMNTNSSHRRVMKVTPMSIINLEMFANVMNMSYSIVSVLLNSRVQK

**SEQ ID NO: 18** (OR20 from *Acyrthosiphon pisum)*

MRSSSATVVDVMLFKAIGLYQLLCPADRGGYSVRSRRALMTALGLSFALHSFQVPYLYYALNDL
QRFAYMAAVIIYGMMCSFKGYVLVTNADRLWLVLNAADYGYTGCGHRDPSRLRRCRATLSALLR
TFVALSYGTLIVWIVLPFFVDEYTGITNSDGTVTRYRTTIHNMQYPIPLAVYNSRPVWALIYVT
ELYVCIVNVFIWSLFDCYLVTMCFVLNAQFHTMSAGYGTLGIRRTGSSPPDTTFAGVRRIKFDE
IESNHYSDLISHIQDNQNLIKMFDVFFEVVRPVVLVQIANGSYSVISLIFLTALMYLMGVPVLS
AAFLKFICGLISLTIELFIFCYGFNHIETAKSVLNFGIYSSNWTEMDLTFKKTMLLTMKMNSSH
KRAMKVSPNSAVGLEMFARVMNMSYSTVSVLLNSRS

**SEQ ID NO: 19** (OR22c from *Acyrthosiphon pisum)*

FKHQGLVADLLPNIRVMQGVGHFMFNYYSEGKKFPHRIYCIVTLLLLLLQYGMMAVNLMMESDD
VDDLTANTITMLFFLHPIVKMIYFPVRSKIFYKTLAIWNNPNSHPLFAESNARFHALAITKMRR
LLFCVAGATIFSVISWTGITFIEDSPIPRLMIRTFYPFNAMSGAGHVFALIYQFYYLVISMAVS
NSLDVLFCSWLLFACEQLQHLKAIMKPLMELSATGLTKKQEMLVRSAIKYWVERHKHVVRLVTA
VGDAYGVALLLHMLTTTITLTLLAYQATKVNGVNVYAATVIGYLLYTLGQVFLFCIFGNRLIEE

SSSVMEAAYSCHWYDGSEEAKTFVQIVCQQCQKAMSISGAKFFTVSLDLFASVLGAVVTYFMVL
VQLK

**SEQ ID NO: 20** (OR23 from *Acyrthosiphon pisum)*

MNLNDEQNYIVNLKLMKITGFYHLISSRAPKYFGFNVYKVTAAIEVMTGIFSIIMLFLSSYYYL
DNTNELMSHFMLVVAIFFSTLKIFWVSRNSETIWNNMDMTCINFLSYTGHKKEILKKARAKSIS
TTILFVILWSSVTVAWSISPFFVKDVYLNIKFKDETRRFRYNSLNYVYPISEEFYNEHFLYFYV
VEMLSVVFWGHGTVAYDTFVISICITIAFQLKTIAVSYISLNDKKGDIKNLKDNDLEAMFNLKL
LIQDQQNMFKKIKEIYKIFEPVTFVQLAAQSMLIIILQAYMIFINHYNGFSLLSVPIIKLIVTVA
PNIIHLFITCYLYTNINHQQDSMNFALYSSDWTAMSINYKKMLLFTMRMNDAEKLKLKISLRKI
VNLEMFASVMHLTYSIISVLAKSYGNTNTK

**SEQ ID NO: 21** (OR25 from *Acyrthosiphon pisum)*

MATGIKTVSKNEDNFMINMRLMKKTGFYQLLDSRSLKVFGHNVFKCMSVVQMSILSSVAFIFVA
NIYYFSDDINTVMMYSMLITSDVLSILKLYYILQNSDTIWNCIQMTSIDDLSYKYHDRRILEEG
RSKSTSYSILIMFMWLNLIVSWSLGPLFVTNYFLIVEQNDEIYRYRFNIMNFAFPATDRFYNDN
FMIYYGIEFITLVLWCHCTMNFDVLLLSMNITFKYQLKTISNSFSAFNFTRYNDFKNNRTKNVK
HHKESESMFDFKSLIYDQQRVIENMKNIYRVFRPVVLTQLASESLIIMLLSCIIMLNYFNGISL
LSALNLRIFAAISTFLFHIYVICYLFDDVNEQKDSMNLALYSSDWTTSDLQHKILLLHAMRMNN
AENLRLQVTRNKIVNFQMFTYVRMIFFSFYYSGYCGHYFY

**SEQ ID NO: 22** (OR31 from *Acyrthosiphon pisum)*

MNPTFKHFFKGDCTNITKPSPMETCIDHTCTINLNILKQCGFYQIFDPNSKKIFGWNVYRISFI
ALTVITQCLIGFGNCGFLFELEDTTDNIDLFLIIFSNSYFCLTEWKVVILIINRKKFLELLDVT
DLIFLKSKQCRKNIKILCKHRIRALQLTNLYFKFCIFVIIEWIIFPIMINSFIAHKTENRRLEN
VVNRRYPVDVNTYNKYYILFYVFEIIIGVKTVYLVLMVDILLLSIGWAIVIQYEVLAEAFKNIG
YNENLQKDHDHDVDDYKYFKSILFDQQQLDSKVKLYFPIVKPIVLMHVAINSVLFIMLSNSFLM
VFLSTESFTYKIVNLFKIGTGILYICLQLFLYCHLFDNINLKRKSVNLGIYSCNWTKMDLKFKK
LLLLTMQINDANYITIKASTKTIVNLPIFANVLMTSYNIVSVMVKTMSKYRKT

**SEQ ID NO: 23** (OR37 from *Acyrthosiphon pisum)*

MKWLQDHEVAINLALFKRYQFYQIFNPNGSKLLNYDTYKLTNVMFIVAVTTYNIFSAMCFFTDT
IDTIDSVDLLLMIFIYSIIIISLLKISVLLFNADQIWELFDLTRFDFLTSRQCRKNVGILCKYR

DRSITITNLYQNYSTMVFIIWMITPLVLNTFVVVGGPNQRYHNIFNMQYPVSANIYNQYYYLFY
LMEIAMGIFVLNYSMIVDNFLISLCWVIIAQYEVITTAFEKIGNDCELTTLQNEKNNNSFEAYE
DLKSILMDQNKLYIKLKSFYRVVWIIVIFLIIIDSVLLIILTYSFVMICSSAESFSIFNILKIS
TAFFVFVIQLYLYCYLFDVLNDKKESVNFGLYCCDWTKMDLRFKKLLLLATKFNNANTLKIKST
PNKIVNLQLFSSVMTTAFNIVTVMLKTMNGKN

**SEQ ID NO: 24** (OR38 from *Acyrthosiphon pisum)*

MGIDNMSSLKSNEVAINLKLFKVFRFYHIFDPNSGKLCKFNVYHLAWYIINCVIGCILIYGLLG
YFTEMEDVIDSIFHIQIMFCYLLYSLSLLKIITFLYKANNIWDLLRVTRINFLTSTQCQAHIGI
LHKHRNKSIKITNLISGFAIVTTLEWILFPLVLRLLSKTDASHSNKRFENIFNFRFPVTVCEYN
NYYFIFYIMESFIAIFMLYAYVVTDVFFISVCYVIIAQYEIIKRAYEIVNCEQTSENNNENKNH
NNIIVNDCCDDLISIVMDQQNHYAKLRLFYSTYKLIIVSTVVINSGSIIILTYASVVIFTSPET
IPILSIVKLISAFTYMFFVLFFLCYLMECINNKIESVQLGMYSCNWTAMNIKSKKLLLFSMRMH
NANKLMIKTTPNNIINLQLFNSVMMTSYNIVSAMVNTRSK

**SEQ ID NO: 25** (OR39 from *Acyrthosiphon pisum)*

MFSCDFINRTVNMNSENLFNGGSVAFNLSTYKQLGYYQLLDPKGPHIYGYHLYRTILKIFLLIV
QFITIFGVMGFFIEMEDTDPGKSNSFELIIIILTNCSLSSLKIYTLISNSKIIWDLFDLTRIDFL
RCSRHSKLITKNFVKRCKKSTTITKWIARSFLVGLILWLMGPFIANEEHTEPNTVHRHKNIINI
KFPVTMKTYNNYYFVFYLMEVAVGFCIVYGSVLIDAYLMSFCWIISAQYQSVTKAFATFGYNKQ
GSPKDIYKDFKSIIIDHQNIYLKMKSFYAVVRPITLIHVFAYSCSLIMYAYVIVTIFNSKELFI
IAEIMKIVMTVSNVTMEVFIFCYLFELIDNKKEDVNFGLYSCNWTGMDIKFKQLLLMSMKMNNA
NRFKLKASPDVTINRPFFANVIHTCFKIVSVLIQTQSIDLLN

**SEQ ID NO: 26** (OR42 from *Acyrthosiphon pisum)*

MPNSSEECVMSSSMAKCTGLHYIIDPEGPTVGGHNVFHVTVMVMIGFTVVCLSMCPFGLYYWAN
DVTQCIFLLIYIVNFSFGCFKAFTLVRHSDDICRCLDVTRFDFSSGAIMSDPDSARFFRKCRDA
SSTFTGWFAASSHFVLLVWTLLPFVVVGKGVEINNRDGSTSYYHFNPYNMYFLVSSETYNRLHL
VFHLVEWAFGLCFVLIMVAFDTFMVTLCVAITCQMRGIGNAYSKLGHDRCATASNVCSDGGIES
NKSNNEYLRDLKLIIKDHQAVLGKMNDFYKIVGPVILPQLIVASFTIIFVSFIITRNYFNGMLL
TSTQSLKMCCFPIFFYQVYYTCHAFGNLSHRKNVMNFALYSSDWTQMEIKFKKLLLLAMQMHDA
NKLDMKLTDKLVINLELFTRVINMCYSIFSVLVNSQLKIADKQ

**SEQ ID NO: 27** (OR43 from *Acyrthosiphon pisum)*

MDSKQEKQYIFNMKLARIMGLYQILFPNSTSFFGYNIYHVVTVFFVSFTFAISMLFPIGLLYLR
NDIIAIMYYMGCISNFLLSCFKMVNILYHSKDIWKCIDVTSFNYILYKHYDRNVFKNWQTRSIR
ITYIYIVIALFAFFCWIFSPCIMNKSVIAIRNIDGSYSKYRMNIFNLYLIASNETYNKNFYIFY
VIEIIISICYVYFTIVFDVLMLLVCFAISYQLETISNTIKSLGHEIYTRDNIRSGNSIKLKEKH
GILYNDLITIMTDHQNVLKKLNDFYNIFRSITLTQIFIASSSHVFIWFIAAMSIDEGDNADSIL
SFKLFIVLPLINFQLFMTCSLFGTINEKKDSIIFALYSSNWTNMDLKSKKMILFNLTINNASQL
KMKFTNTKIVNLEMFSHTMRFCYSIFSMLINYNKNKMK

**SEQ ID NO: 28** (Forward primer for site-directed mutagenesis (thrombin cleavage site insertion) of OBP6-His6)
cgcggcagcGCTGGGTACGATAGAACATG
**SEQ ID NO: 29** (Reverse primer for site-directed mutagenesis (thrombin cleavage site insertion) of OBP6-His6)
cggcaccagCGGATCCGGACTCTTGTC

## Claims

**1.** A biosensor for detecting an analyte in a sample, the biosensor comprising:

a protein having an amino acid sequence as defined in SEQ ID NO: 6, or a variant thereof, wherein the variant has at least 80% overall sequence identity to SEQ ID NO: 6; and
a signal generator, wherein the signal generator is configured to output a signal when the analyte is bound to the protein.

**2.** A biosensor according to claim 1, wherein the biosensor further comprises:

a flow path for moving the sample;
a substrate; and

a protein-containing layer immobilised to the substrate and in contact with the flow path, wherein the protein-containing layer comprises the protein.

**3.** Use of a biosensor according to claim 1 or claim 2, wherein the biosensor is used to identify olfactory ligands; or wherein the biosensor is used in high-throughput screening; or wherein the biosensor is used to detect field populations of aphids.

**4.** An in vitro method for detecting an analyte in a sample comprising:

a. providing a biosensor according to claim 1 or claim 2;
b. contacting the biosensor with the sample; and
c. comparing a magnitude of the signal generated by the biosensor when the sample is present with a reference magnitude of the signal generated by the biosensor when the sample is absent.

**5.** A method according to claim 4, wherein the biosensor is used to identify olfactory ligands; or wherein the biosensor is used in high-throughput screening; or wherein the biosensor is used to detect field populations of aphids.

## Patentansprüche

**1.** Biosensor zum Detektieren eines Analyts in einer Probe, der Biosensor umfassend:

ein Protein mit einer Aminosäuresequenz, wie in SEQ ID NO: 6 definiert, oder einer Variante davon, wobei die Variante mindestens 80 % Gesamtsequenzidentität mit SEQ ID NO: 6 aufweist; und
einen Signalgenerator, wobei der Signalgenerator konfiguriert ist, ein Signal auszugeben, wenn der Analyt an das Protein gebunden ist.

**2.** Biosensor nach Anspruch 1, wobei der Biosensor ferner umfasst:

einen Fließweg zum Bewegen der Probe;
ein Substrat; und
eine proteinhaltige Schicht, die an das Substrat immobilisiert ist und mit dem Fließweg in Kontakt ist, wobei die proteinhaltige Schicht das Protein umfasst.

**3.** Verwendung eines Biosensors nach Anspruch 1 oder Anspruch 2, wobei der Biosensor verwendet wird, olfaktorische Liganden zu identifizieren; oder wobei der Biosensor bei Screening mit hohem Durchsatz verwendet wird; oder wobei der Biosensor verwendet wird, Feldpopulationen von Blattläusen zu detektieren.

**4.** In-vitro-Verfahren zum Detektieren eines Analyts in einer Probe, umfassend:

a. Bereitstellen eines Biosensors nach Anspruch 1 oder Anspruch 2;
b. Inkontaktbringen des Biosensors mit der Probe; und
c. Vergleichen einer Größe des durch den Biosensor erzeugten Signals, wenn die Probe vorhanden ist, mit einer Referenzgröße des durch den Biosensor erzeugten Signals, wenn die Probe nicht vorhanden ist.

**5.** Verfahren nach Anspruch 4, wobei der Biosensor verwendet wird, olfaktorische Liganden zu identifizieren; oder wobei der Biosensor bei Screening mit hohem Durchsatz verwendet wird; oder wobei der Biosensor verwendet wird, Feldpopulationen von Blattläusen zu detektieren.

## Revendications

**1.** Biocapteur destiné à détecter un analyte dans un échantillon, le biocapteur comprenant :

une protéine présentant une séquence d'acides aminés telle que définie dans SEQ ID NO : 6, ou un variant de celle-ci, dans lequel le variant présente au moins 80 % d'identité de séquence globale avec SEQ ID NO : 6 ; et
un générateur de signal, dans lequel le générateur de signal est configuré pour émettre un signal lorsque l'analyte est lié à la protéine.

**2.** Biocapteur selon la revendication 1, dans lequel le biocapteur comprend en outre :

un trajet de flux destiné à déplacer l'échantillon ;
un substrat ; et
une couche contenant une protéine immobilisée sur le substrat et en contact avec le trajet de flux, dans lequel la couche contenant une protéine comprend la protéine.

**3.** Utilisation d'un biocapteur selon la revendication 1 ou la revendication 2, dans lequel le biocapteur est utilisé pour identifier des ligands olfactifs ; ou dans lequel le biocapteur est utilisé pour un criblage à haut débit ; ou dans lequel le biocapteur est utilisé pour détecter des populations de terrain de pucerons.

**4.** Procédé in vitro destiné à détecter un analyte dans un échantillon comprenant :

a. la fourniture d'un biocapteur selon la revendication 1 ou la revendication 2 ;
b. la mise en contact du biocapteur avec l'échantillon ; et
c. la comparaison d'une amplitude du signal généré par le biocapteur lorsque l'échantillon est présent avec une amplitude de référence du signal généré par le biocapteur lorsque l'échantillon est absent.

**5.** Procédé selon la revendication 4, dans lequel le biocapteur est utilisé pour identifier des ligands olfactifs ; ou dans lequel le biocapteur est utilisé dans un criblage à haut débit ; ou dans lequel le biocapteur est utilisé pour détecter des populations de terrain de pucerons.

FIG. 1

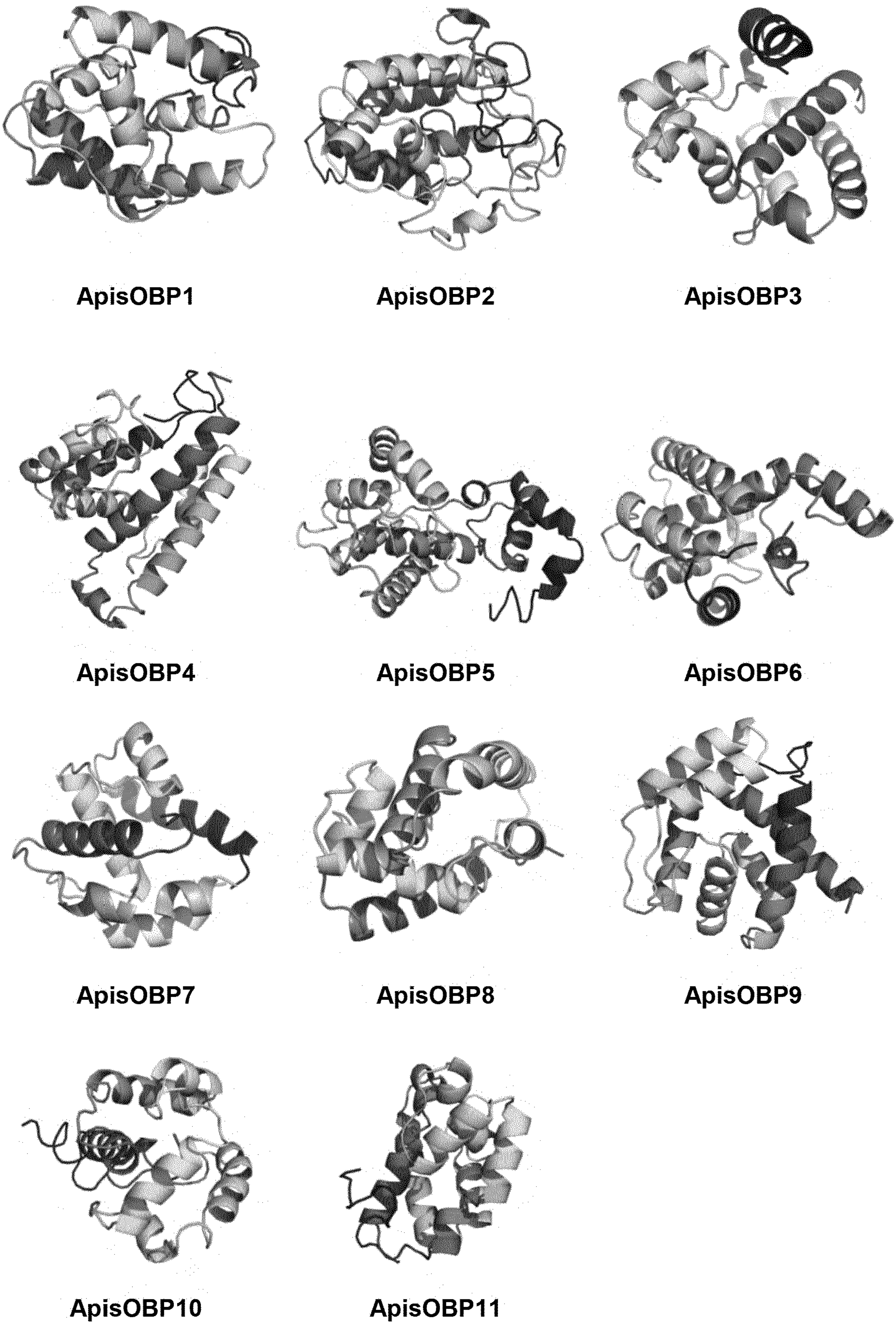
ApisOBP1
ApisOBP2
ApisOBP3
ApisOBP4
ApisOBP5
ApisOBP6
ApisOBP7
ApisOBP8
ApisOBP9
ApisOBP10
ApisOBP11

FIG. 2

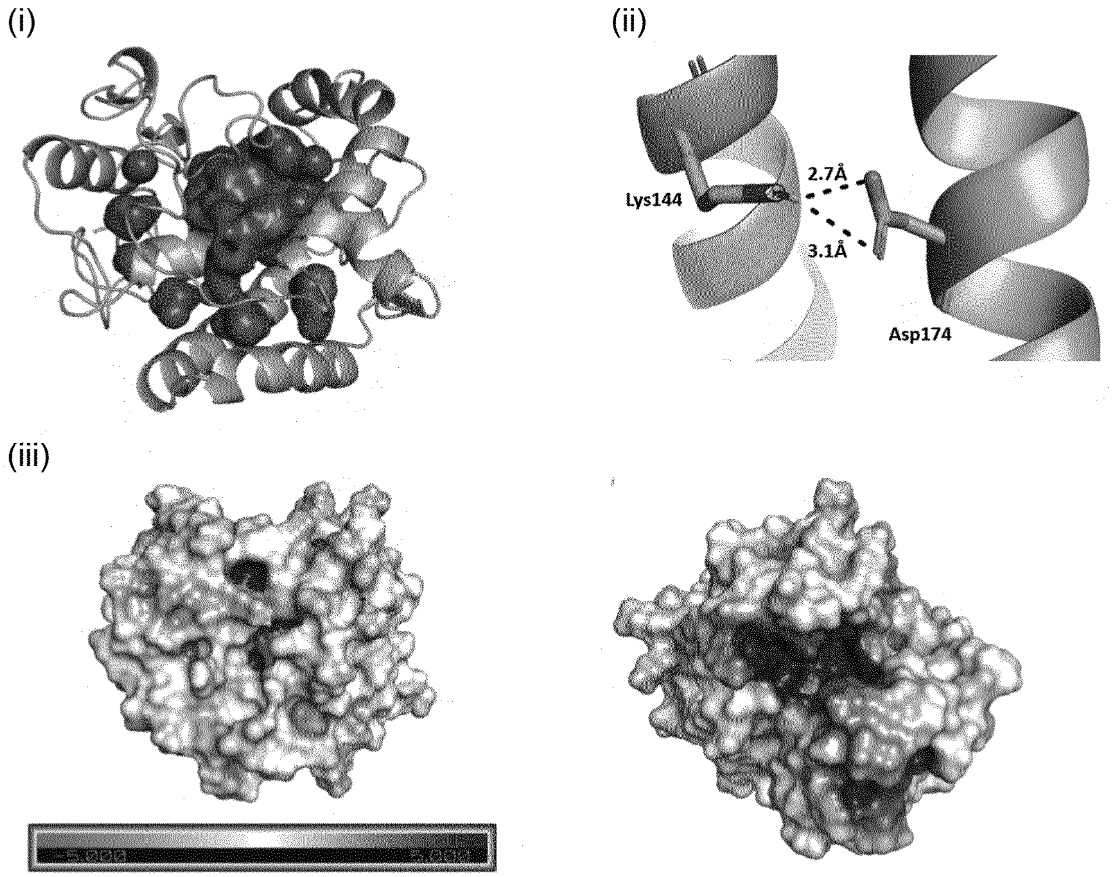

FIG. 3

| Ligand | A. Pisum OBP | | | | | | | |
|---|---|---|---|---|---|---|---|---|
| | OBP6 | | OBP7 | | OBP8 | | OBP9 | |
| | Binding energy (kcal $mol^{-1}$) | $K_i$ (µM) | Binding energy (kcal $mol^{-1}$) | $K_i$ (µM) | Binding energy (kcal $mol^{-1}$) | $K_i$ (µM) | Binding energy (kcal $mol^{-1}$) | $K_i$ (µM) |
| (1*R*,4a*S*,7*S*,7a*R*)-nepetalactol | -7.67 | 2.37 | -5.99 | 40.71 | -5.89 | 48.44 | -5.31 | 129.21 |
| (4a*S*,7*S*,7a*R*)-nepetalactone | -7.52 | 3.07 | -6.03 | 37.41 | -6.15 | 31.26 | -5.79 | 57.24 |
| (1*S*,4a*R*,7*R*,7a*S*)-nepetalactol | -7.69 | 2.30 | -6.00 | 39.68 | -6.38 | 20.89 | -5.47 | 97.43 |
| (4a*R*,7*R*,7a*S*)-nepetalactone | -7.60 | 2.69 | -5.98 | 41.91 | -6.33 | 22.92 | -5.71 | 65.65 |
| (*E*)-β-farnesene | -6.74 | 11.50 | -6.76 | 11.18 | -6.02 | 38.88 | -5.08 | 188.75 |
| (*S*)-germacrene D | -3.12 | 5.14 | -6.65 | 13.31 | -7.32 | 4.30 | -6.07 | 35.79 |
| (1*R*,4*E*,9*S*)-Caryophyllene | -6.71 | 12.00 | NA | NA | -7.47 | 3.36 | -6.40 | 20.27 |
| Myrcene | -6.34 | 22.41 | NA | NA | -4.71 | 355.75 | -4.41 | 584.36 |
| (*E*)-Ocimene | -6.44 | 19.06 | NA | NA | -4.96 | 230.07 | -4.51 | 494.01 |
| (4*R*)-linalool | -6.61 | 14.26 | NA | NA | -4.83 | 286.76 | -4.62 | 412.93 |
| (4*S*)-linalool | -6.56 | 15.58 | NA | NA | -4.84 | 285.02 | -4.73 | 343.23 |
| (*Z*)-jasmone | -7.69 | 2.31 | NA | NA | -5.99 | 40.90 | -7.69 | 2.31 |

NA = no favourable docking conformations were found in the screening

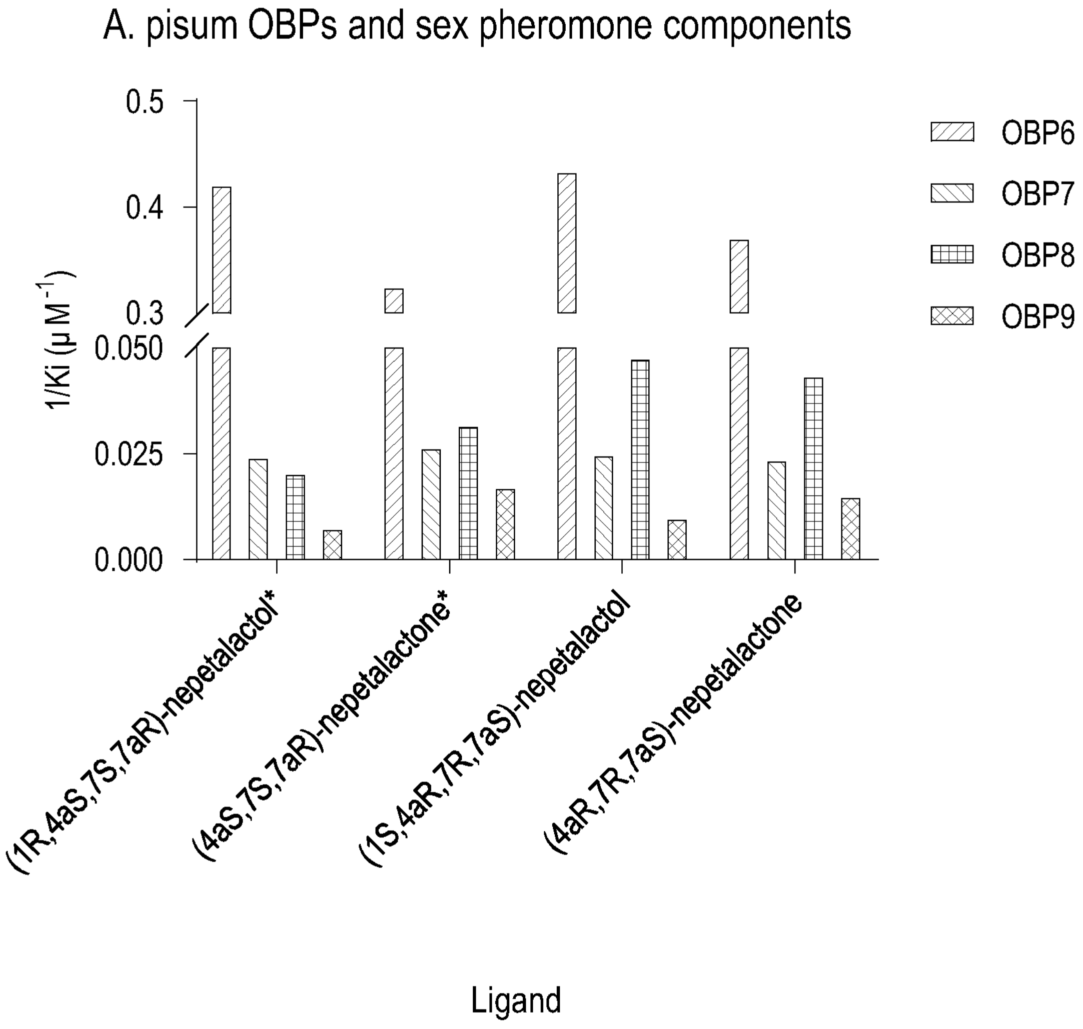
A. pisum OBPs and sex pheromone components
1/Ki (μM-1)
0.5
0.4
0.3
0.050
0.025
0.000
OBP6
OBP7
OBP8
OBP9
(1R,4aS,7S,7aR)-nepetalactol*
(4aS,7S,7aR)-nepetalactone*
(1S,4aR,7R,7aS)-nepetalactol
(4aR,7R,7aS)-nepetalactone
Ligand

Fig. 4

FIG. 5

(i)

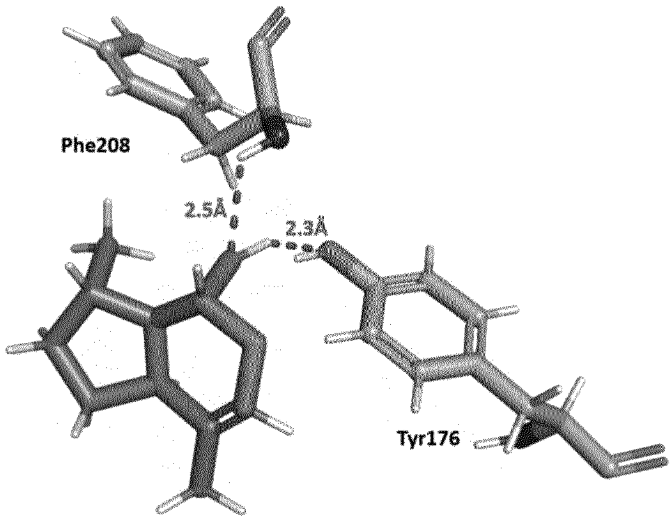


(ii)

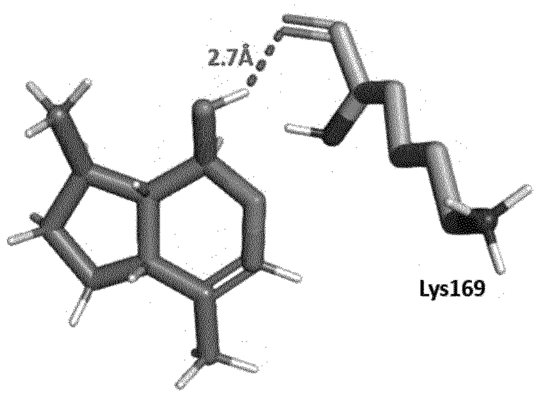

FIG. 6

(i)

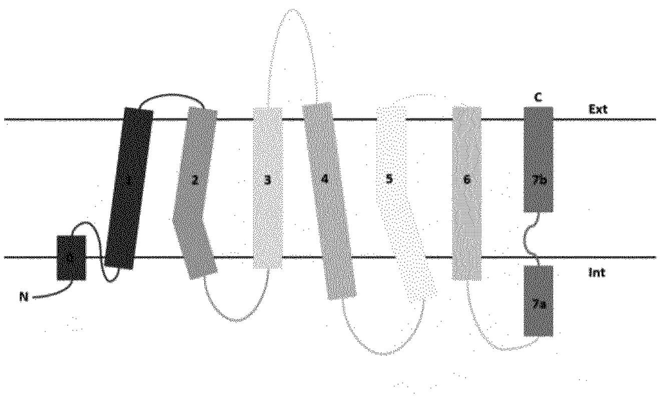


(ii)

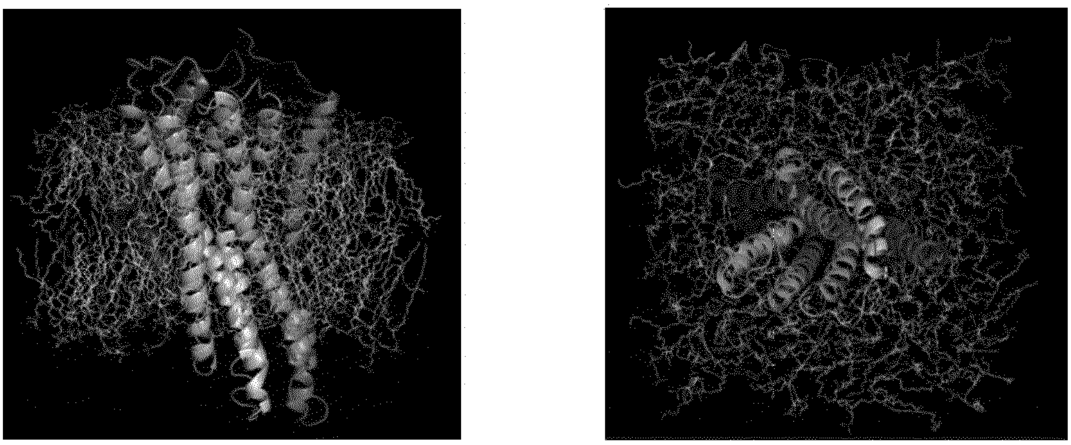

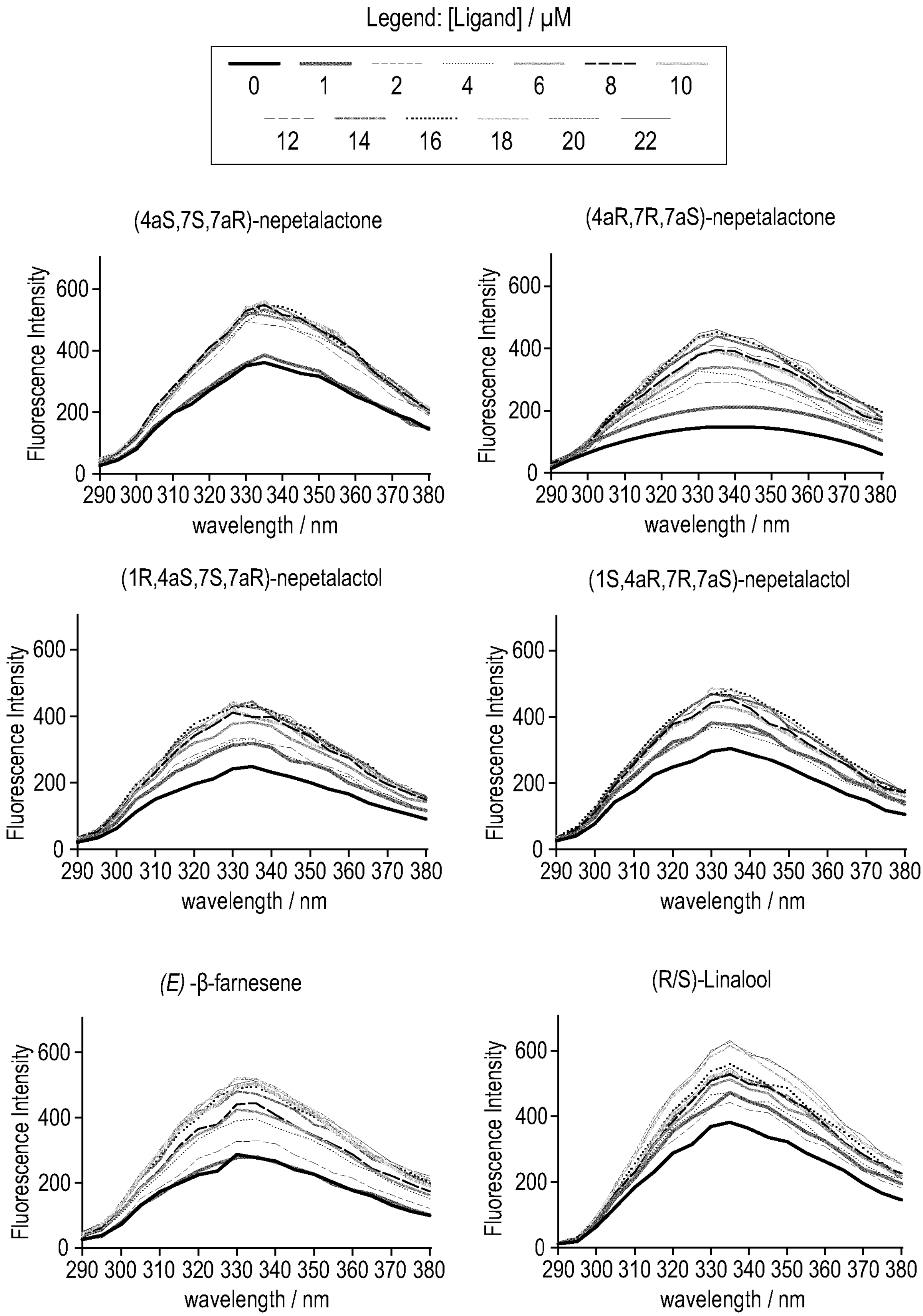
Legend: [Ligand] / µM
0
1
2
4
6
8
10
12
14
16
18
20
22
(4aS,7S,7aR)-nepetalactone
(4aR,7R,7aS)-nepetalactone
(1R,4aS,7S,7aR)-nepetalactol
(1S,4aR,7R,7aS)-nepetalactol
(E) -β-farnesene
(R/S)-Linalool
Fluorescence Intensity
wavelength / nm
0
200
400
600
290 300 310 320 330 340 350 360 370 380

Fig. 7

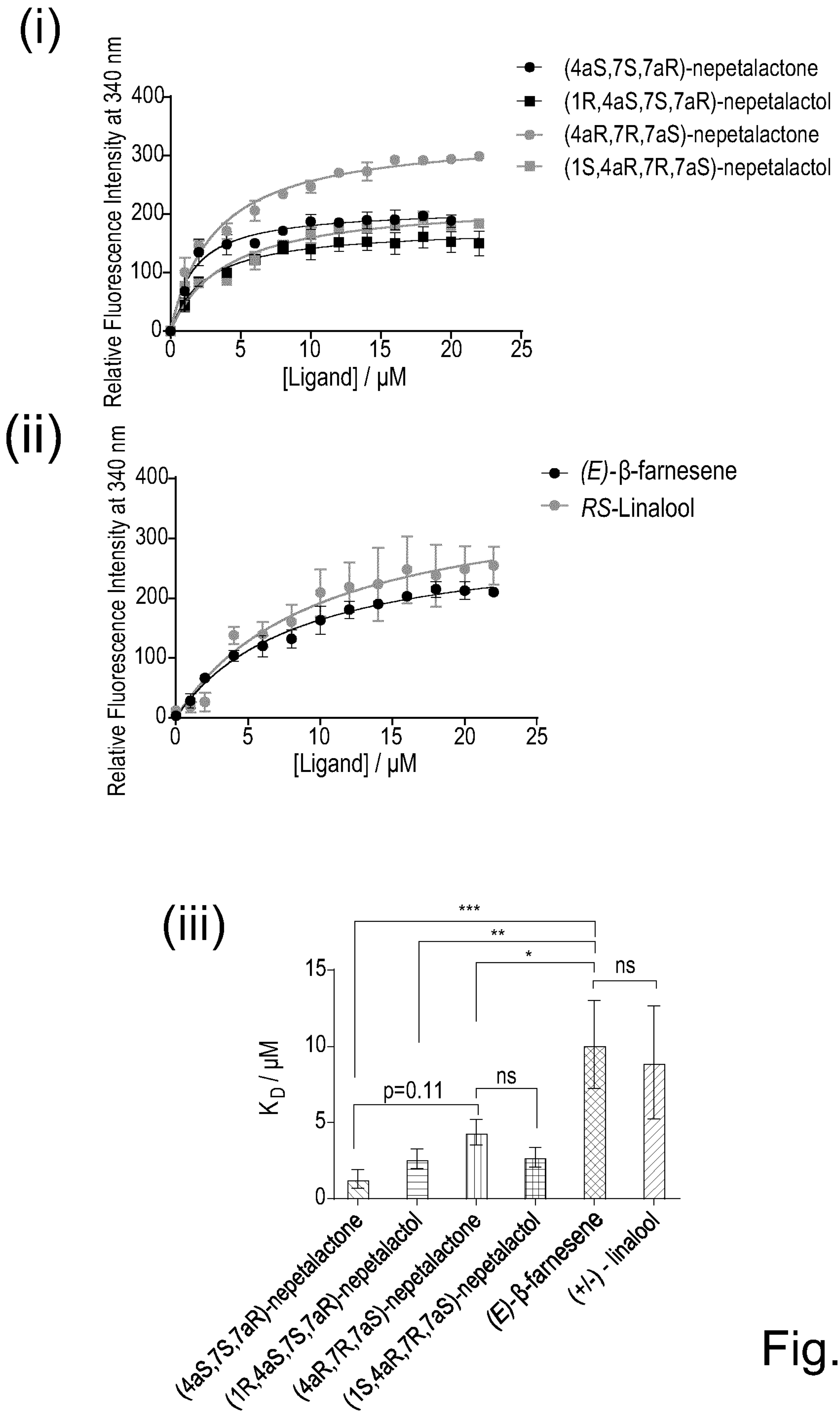
(i)
Relative Fluorescence Intensity at 340 nm
400
300
200
100
0
0
5
10
15
20
25
[Ligand] / µM
(4aS,7S,7aR)-nepetalactone
(1R,4aS,7S,7aR)-nepetalactol
(4aR,7R,7aS)-nepetalactone
(1S,4aR,7R,7aS)-nepetalactol
(ii)
Relative Fluorescence Intensity at 340 nm
400
300
200
100
0
0
5
10
15
20
25
[Ligand] / µM
(E)-β-farnesene
RS-Linalool
(iii)
***
**
*
ns
15
10
5
0
K_D / µM
p=0.11
ns
(4aS,7S,7aR)-nepetalactone
(1R,4aS,7S,7aR)-nepetalactol
(4aR,7R,7aS)-nepetalactone
(1S,4aR,7R,7aS)-nepetalactol
(E)-β-farnesene
(+/-) - linalool

Fig. 8

FIG. 9

| Ligand | *In silico* $K_D$ / µM | Fluorescence $K_D$ / µM |
|---|---|---|
| (1*R*,4a*S*,7*S*,7a*R*)-nepetalactol | 2.37 | 2.62 ± 0.63 |
| (4a*S*,7*S*,7a*R*)-nepetalactone | 3.07 | 1.30 ± 0.60 |
| (1*S*,4a*R*,7*R*,7a*S*)-nepetalactol | 2.30 | 2.65 ± 0.80 |
| (4a*R*,7*R*,7a*S*)-nepetalactone | 2.69 | 4.37 ± 0.81 |
| (*E*)-β-farnesene | 11.50 | 10.12 ± 2.88 |
| Linalool* | 14.26 | 8.95 ± 3.71 |

*For *in silico* (*R*)-linalool is reported, for fluorescence (*R/S*)-linalool is reported

FIG. 10

| Ligand | ApisOBP1 | | ApisOBP2 | | ApisOBP3 | | ApisOBP4 | | ApisOBP5 | | ApisOBP10 | | ApisOBP11 | |
|---|---|---|---|---|---|---|---|---|---|---|---|---|---|---|
| | Binding energy (kcal $mol^{-1}$) | $K_i$ (µM) | Binding energy (kcal $mol^{-1}$) | $K_i$ (µM) | Binding energy (kcal $mol^{-1}$) | $K_i$ (µM) | Binding energy (kcal $mol^{-1}$) | $K_i$ (µM) | Binding energy (kcal $mol^{-1}$) | $K_i$ (µM) | Binding energy (kcal $mol^{-1}$) | $K_i$ (µM) | Binding energy (kcal $mol^{-1}$) | $K_i$ (µM) |
| (1*R*,4a*S*,7*S*,7a*R*)-nepetalactol | -5.85 | 51.09 | 8.79 | NA | -6.14 | 31.33 | -5.94 | 44.08 | -4.28 | 725.14 | 1.26 | NA | -5.92 | 46.07 |
| (4a*S*,7*S*,7a*R*)-nepetalactone | -6.60 | 14.47 | 2.33 | NA | NA | NA | -5.86 | 50.36 | -5.00 | 214.56 | 2.60 | NA | -6.44 | 18.94 |
| (1*S*,4a*R*,7*R*,7a*S*)-nepetalactol | -6.05 | 37.0 | 10.78 | NA | NA | NA | -6.45 | 18.55 | -4.24 | 784.16 | 10.06 | NA | -5.94 | 43.96 |
| (4a*R*,7*R*,7a*S*)-nepetalactone | -6.53 | 16.37 | 10.1 | NA | NA | NA | -6.17 | 30.01 | -5.07 | 192.32 | NA | NA | -6.39 | 20.67 |

NA = no favourable docking conformations were found in the screening

FIG. 11

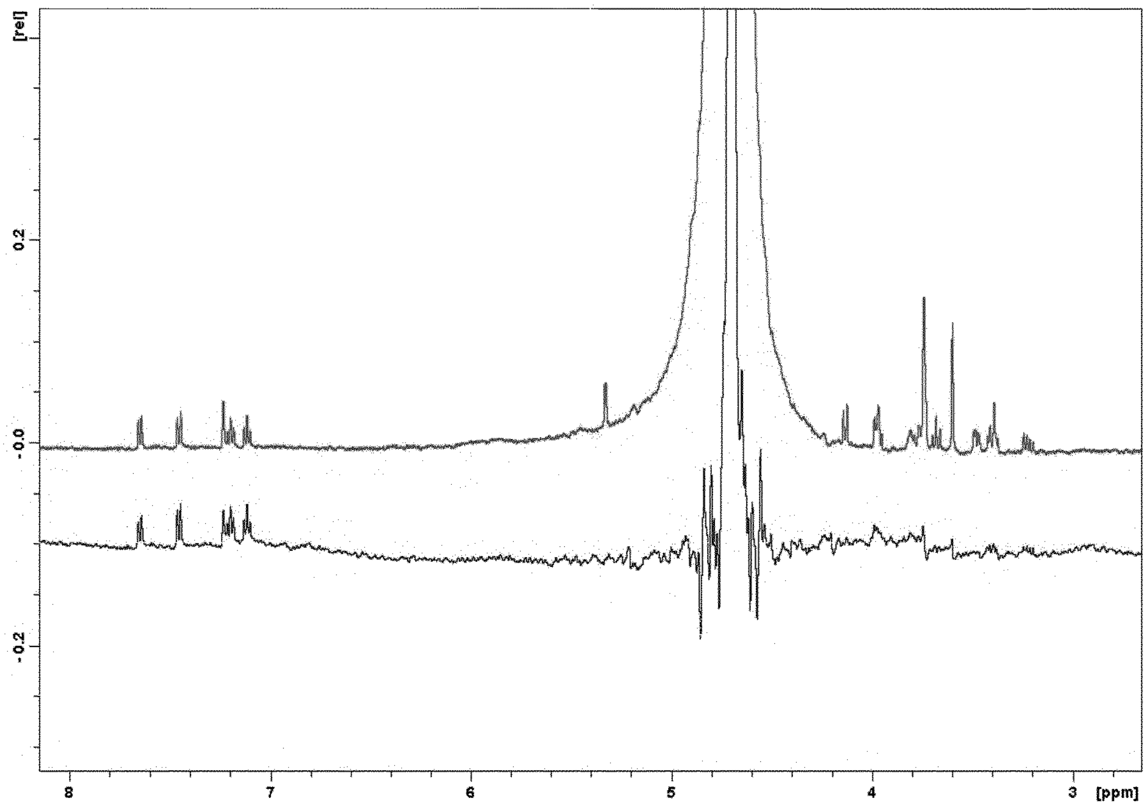


FIG. 12

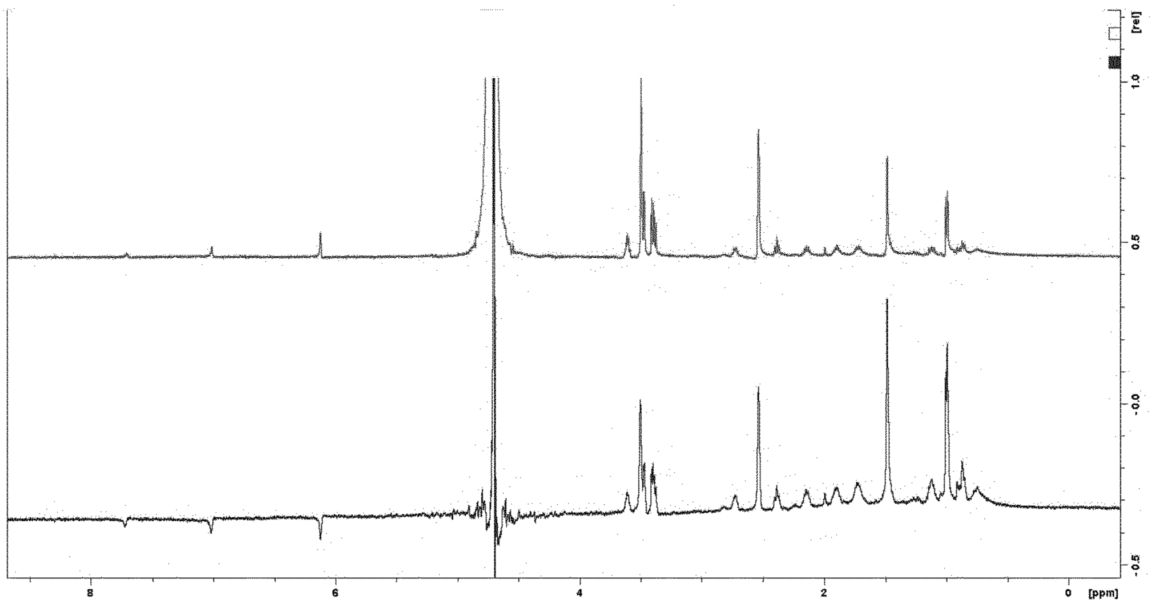

(i)

| δ / ppm (multiplicity) | Assignment | Epitope Mapping |
|---|---|---|
| 1.21 (d) | 9-H | 110 % |
| 1.50-1.59 (m) | 5-H | 75 % |
| 1.64 (s) | 4-H | 100 % |
| 1.89-1.98 (m) | 6-H or 7-H | 90 % |
| 2.02-2.11 (m) | 6-H or 7-H | 60 % |
| 2.31-2.39 (m) | 8-H | 70 % |
| 2.05 (q) | 10-H | 50 % |
| 2.71 (s) | d-DMSO | N/A |
| 6.18-6.20 (m) | 2-H | -25 % |

(ii)

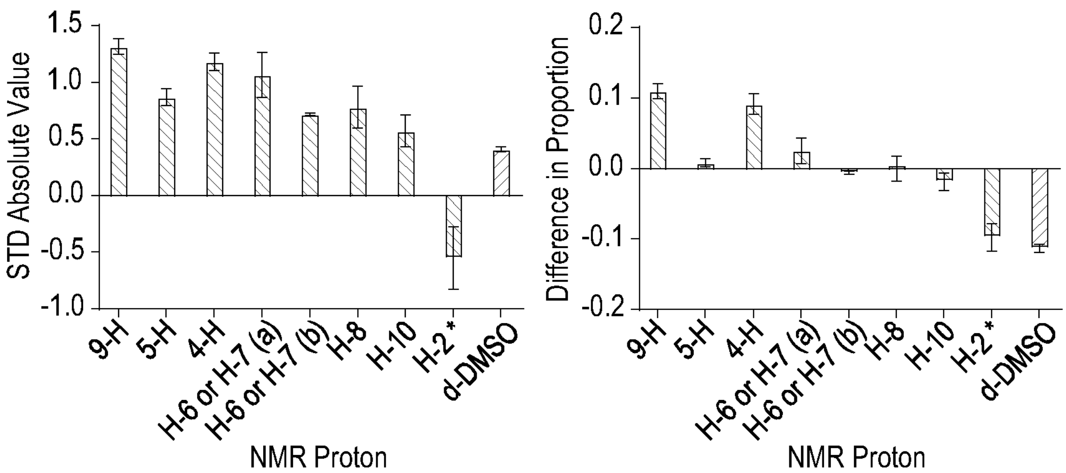


(iii)

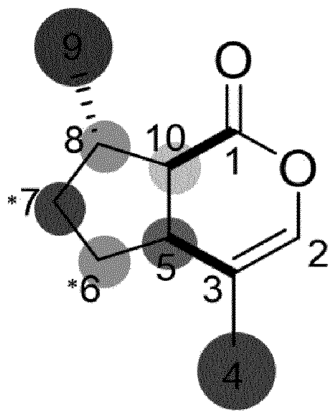

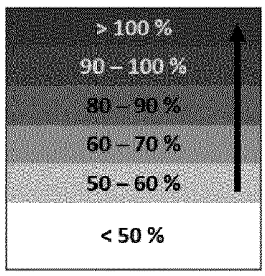


Fig. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SUN et al.** New Analogues of (E)-β-Farnesene with Insecticidal Activity and Binding Affinity to Aphid Odorant-Binding Proteins. *Journal of Agricultural and Food Chemistry*, 2011, vol. 59 (6), 2456-2461
- **BIASIO et al.** Expression pattern analysis of odorant-binding proteins in the pea aphid Acyrthosiphon pisum. *Insect Science*, 2015, vol. 22 (2), 220-234
- **PELOSI et al.** Odorant-Binding Proteins as Sensing Elements for Odour Monitoring. *Sensors*, 2018, vol. 18 (10), 1-19
- **PELOSI et al.** From radioactive ligands to biosensors: binding methods with olfactory proteins. *Applied Microbiology and Biotechnology*, 2018, vol. 102 (19), 8213-8227
- **DAWSON et al.** *Bioorganic Med. Chem.*, 1996, vol. 4 (3), 351-361
- **SCHREIBER et al.** *J. Am. Chem. Soc.*, 1986, vol. 108, 8274-8277